# EUROPEAN PATENT APPLICATION

(11) **EP 3 988 550 A1**
(43) Date of publication of application: **27.04.2022**
(21) Application number: 20827795.4
(22) Date of filing: 22.06.2020
(51) Int. Cl.: C07D 473/34, C07H 19/167, A61K 31/7076, A61P 37/06

(54) **6-HYDRAZINOADENOSINE COMPOUND HAVING A2A ADENOSINE RECEPTOR AGONIST ACTIVITY**

(30) Priority: 21.06.2019 CN 201910542462
(71) Applicant: Academy Of Military Medical Sciences, Haidian District Beijing 100850 (CN)
(72) Inventor: ZHONG, Wu, Beijing 100850 (CN); ZHANG, Min, Beijing 100850 (CN); ZHOU, Xinbo, Beijing 100850 (CN); FAN, Shiyong, Beijing 100850 (CN); LI, Song, Beijing 100850 (CN)
(74) Representative: Weickmann & Weickmann PartmbB
(86) International application number: PCT/CN2020/097427
(87) International publication number: WO 2020/253870

(57) **Abstract**

The present disclosure provides 6-hydrazinoadenosine represented by the general Formula (I) and its derivatives with A_{2A} adenosine receptor agonist activity, and pharmaceutical compositions containing them. The compound and composition can be used as A_{2A} adenosine receptor agonists and used as medicament.

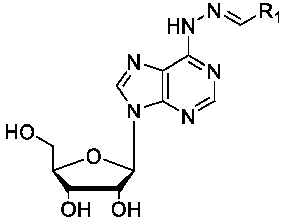

## Description

### Technical Field

The present disclosure belongs to the technical field of medicine, and specifically relates to 6-hydrazinoadenosine and derivatives thereof as an A_{2A} adenosine receptor agonist, and a pharmaceutical composition containing the same. These compounds and composition can be used as medicament.

### Background Art

For drugs for the treatment of central nervous system diseases, one of the main reasons for the failure of their development and related research lies in the obstruction of the blood-brain barrier (BBB), which prevents a drug from being delivered to the central nervous system, and accumulating in the brain to reach an effective dose to produce a corresponding therapeutic effect. Therefore, a key factor for the successful development of drugs that target the center nervous system is to cross the blood-brain barrier. Drug delivery across the blood-brain barrier has been a challenging research area in the past few decades. Researchers have made considerable efforts to develop various drug delivery systems. A series of studies of strategics have revealed that it is very difficult to transport drugs and contrast agents across the blood-brain barrier.

BBB (blood-brain barrier) restricts molecules from entering the brain through two main structural characteristics. First, there are tight junctions (TJs) between cerebral vascular endothelial cells, which seal the endothelial cells and result in low permeability of molecules in blood through the BBB. Second, compared with peripheral vascular endothelial cells, there are few transport pathways between cerebral vascular endothelial cells, but active efflux transporters, such as P-glycoprotein (P-gp) on brain capillary endothelial cells (BCECs), are at a very high expression level.

Considering the key role of TJs in restricting molecules from entering the brain (HUBER J D et al., Trends Neurosci, 2001, 24(12): 719-25), reversibly changing the tightness of TJs may be a feasible way to up-regulate BBB permeability. Temporarily opening TJs is a feasible way of brain drug delivery, and this way has a high passing efficiency for the therapeutic drugs and less limitation in molecular weight. Bynoe et al. demonstrated that the specific agonizing of A_{2A} adenosine receptor (A_{2A}AR) on mouse BCECs could promote brain drug absorption (CARMAN A J et al., J Neurosci, 2011, 31(37): 13272-80).

Further studies have shown that the agonizing of A_{2A} adenosine receptor (A_{2A}AR) can up-regulate BBB permeability and temporarily increase the intercellular space of brain capillary endothelial cells. Studies have shown that the A_{2A}AR signaling pathway modulates intracellular actin to change cytoskeletal elements, which leads to cell morphology contraction, destruction of TJs integrity, and increased barrier permeability (SOHAIL M A et al., Hepatology, 2009, 49(1): 185-94). These studies have greatly expanded the potential application fields and development space of A_{2A}AR agonists. The development of high-efficiency A_{2A}AR agonists is of great significance to the study of strategies for opening the blood-brain barrier (patent CN200980117596.0).

Due to the widespread distribution of A_{2A}AR in the human body, A_{2A}AR agonists can be used to treat various pathological diseases. Adenosine mediates A_{2A}AR to produce potential immunosuppressive and hypotensive effects. One of the main potential therapeutic effects of A_{2A}AR agonists is anti-inflammatory and immunosuppressive effect. It regulates the activity of neutrophils, macrophages and T lymphocytes (DE LERA RUIZ M et al., J Med Chem, 2014, 57(9): 3623-50; VARANI K et al., FASEB J, 2010, 24( 4): 1192-204) to achieve the above functions. From the perspective of cell signaling pathways, the agonizing of A_{2A} reduces the NF-kB pathway, reduces inflammatory cytokines such as tumor necrosis factor α (TNF-α), interleukin-1 β (IL-1β), IL-8, IL-6, and inhibits the release of matrix metalloproteinase-1 (MMP-1) and MMP-3 (HASKO G et al., Nat Rev Drug Discov, 2008, 7(9): 759- 70). Therefore, selective agonists have been developed to treat related diseases, such as allergic rhinitis, asthma, and chronic obstructive pulmonary disease. Furthermore, A_{2A}AR agonists are powerful vasodilators and have been used as diagnostic reagents for cardiac pharmacological stress tests (patent CN200580033215.2). In addition, the further potential therapeutic application of A_{2A}AR agonists is the treatment of psychosis and Huntington's disease (AKKARI R et al., Curr Top Med Chem, 2006, 6(13): 1375-99; BOSCH MP et al., J Med Chem, 2004, 47(16): 4041-53). It has been shown that A_{2A}AR agonists have neuroprotective effects on neurodegenerative disease models by reducing the release of excitatory neurotransmitters, apoptosis and inflammation (MULLER CE et al., Biochim Biophys Acta, 2011, 1808(5): 1290-308; RIVERA-OLIVER M, etc., Life Sci, 2014, 101(1-2): 1-9).

Although A_{2A}AR agonists as described above have been increasingly developed, only one receptor agonist, regadenoson (an adenosine analog), is approved as a coronary vasodilator in the United States. Regadenoson is a selective A_{2A} adenosine receptor agonist jointly developed by CV Pharmaceuticals and Astellas. This product has been marketed in the United States and Europe. It is mainly used as a coronary vasodilator for myocardial perfusion imaging. Therefore, there is still a need in the art for A_{2A} receptor agonists that have novel structure, are effective and optionally have one or more physiological and/or physicochemical advantages, and it is important to continuously synthesize and test additional A_{2A} receptor agonists so as to develop new and improved therapeutic agents.

### Contents of the Invention

The present disclosure provides a new class of small molecule agonists acting on A_{2A} adenosine receptor, which can agonize A_{2A} adenosine receptor, thereby achieving, on the one hand, the purpose for prevention and/or treatment of a human pathological state or symptom, in which the prevention and/or treatment of a human pathological state or symptom is related to the activity of A_{2A} adenosine receptor, and the prevention and/or treatment of a human pathological state or symptom requires agonizing of A_{2A} adenosine receptor; on the other hand, the purpose for increasing the permeability of blood-brain barrier of a subject receiving the therapeutic drug.

The first aspect of the present disclosure provides a compound represented by general Formula (I), or a stereoisomer thereof, or a pharmaceutically acceptable salt of the compound or stereoisomer, or a pharmaceutically acceptable hydrate or solvate of the compound or stereoisomer, or a pharmaceutically acceptable ester of the compound or stereoisomer, wherein the compound has a structure represented by the general Formula (I) as follows: 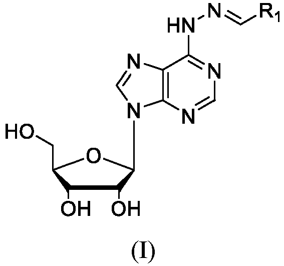 wherein,
R₁ is selected from the group consisting of aryl, heteroaryl, cycloalkyl, C₁₋₁₀ alkyl, C₁₋₁₀ heteroalkyl or C₂₋₁₀ alkenyl;
R₁ is optionally substituted with one or more R', each R' is independently selected from the group consisting of phenyl, halophenyl, amino-substituted phenyl, benzyloxy, halobenzyloxy, phenylamino, heteroaryl, cycloalkyl, heterocycloalkyl, C₁₋₆ alkyl, halogenated C₁₋₆ alkyl, C₂₋₆ alkenyl, C₁₋₆ alkoxy, C₁₋₆ alkylthio, -NHC(O)R¹⁰, halogen or cyano, wherein R¹⁰ is C₁₋₆ alkyl.

In some embodiments, R₁ is selected from C₆₋₁₀ aryl, 5- to 7-membered heteroaryl, 5- to 6-membered cycloalkyl, 5- to 6-membered heterocycloalkyl, C₁₋₁₀ alkyl, C₁₋₁₀ heteroalkyl or C₂₋₁₀ alkenyl.

In some embodiments, R₁ is selected from the group consisting of phenyl, pyrrolyl, imidazolyl, thiazolyl, furyl, pyridyl, cyclopentyl, cyclohexyl, methyl, ethyl, n-propyl, isopropyl, n-butyl, isobutyl, tert-butyl, trifluoromethyl, methoxy, ethoxy, n-propoxy, isopropoxy, n-butoxy, tert-butoxy, sec-butoxy, n-pentyloxy, n-hexyloxy, methylthio, ethylthio, n-propylthio, isopropylthio, n-butylthio, tert-butylthio, sec-butylthio, n-pentylthio, n-hexylthio or C₂₋₁₀ alkenyl.

In some embodiments, R₁ is selected from the group consisting of phenyl, pyrrolyl, furyl, imidazolyl, thiazolyl, cyclohexyl, alkylthio, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, vinyl, or decadienyl.

In some embodiments, R₁ is phenyl. R₁ is optionally substituted with one or more R', each R' is independently selected from the group consisting of phenyl, halophenyl, amino-substituted phenyl, benzyloxy, halobenzyloxy, phenylamino, heteroaryl, cycloalkyl, heterocycloalkyl, C₁₋₆ alkyl, halogenated C₁₋₆ alkyl, C₂₋₆ alkenyl, C₁₋₆ alkoxy, C₁₋₆ alkylthio, -NHC(O)R¹⁰, halogen or cyano, wherein R¹⁰ is C₁₋₆ alkyl.

In some embodiments, R₁ is halopyridyl, such as bromopyridyl, such as 5-bromopyridyl, such as 5-bromopyridin-2-yl.

In some embodiments, R₁ is thiazolyl, such as thiazol-5-yl.

In some embodiments, R₁ is cyclohexyl.

In some embodiments, R₁ is selected from the following groups:

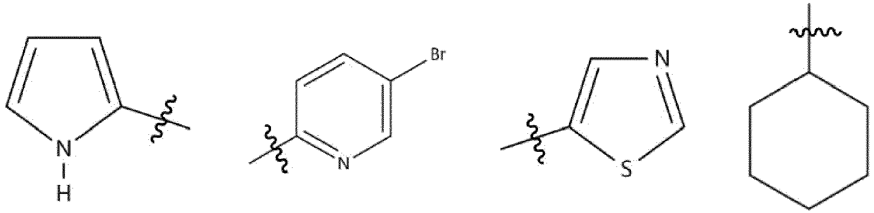

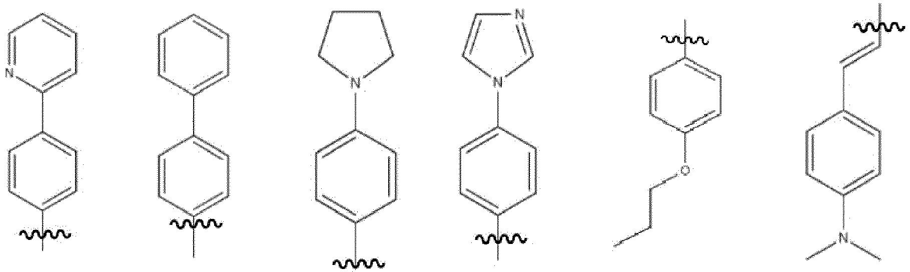

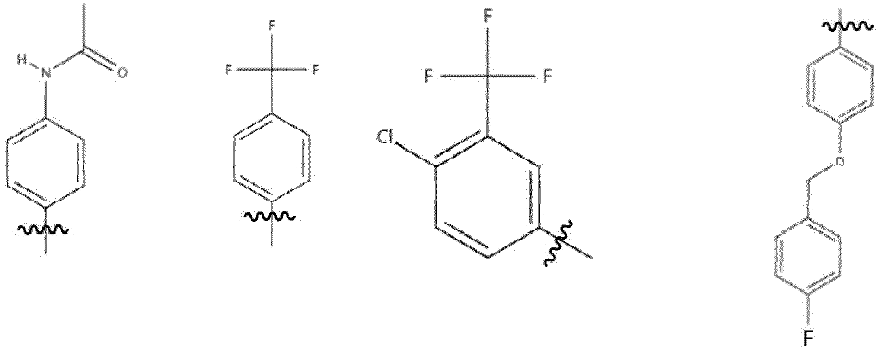

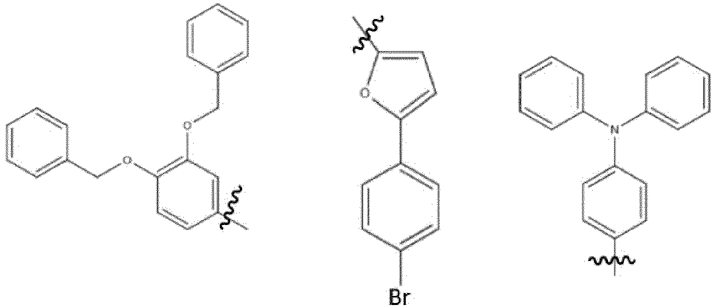

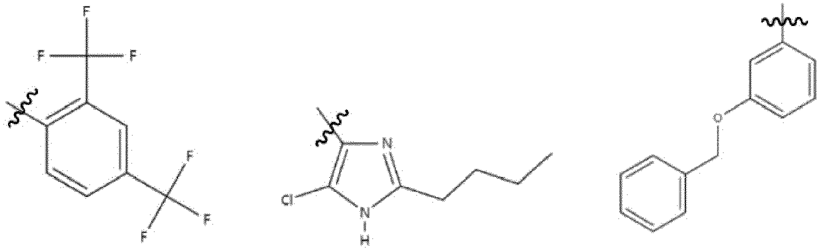

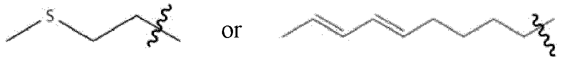

In some embodiments, each R' is independently selected from the group consisting of phenyl, halophenyl, amino-substituted phenyl, benzyloxy, halobenzyloxy, phenylamino, imidazolyl, pyridyl, 5- to 6-membered cycloalkyl, 5- to 6-membered heterocycloalkyl, C₁₋₆ alkyl, C₁₋₆ haloalkyl, -NHC(O)R¹⁰, halogen or cyano, wherein R¹⁰ is C₁₋₄ alkyl;

In some embodiments, each R' is independently selected from the group consisting of phenyl, halophenyl, dimethylamino-substituted phenyl, benzyloxy, halobenzyloxy, diphenylamino, 1H-imidazol-1-yl, pyridin-2-yl, 1H-imidazol-1-yl, pyrrolidin-1-yl, cyclopentyl, cyclohexyl, methyl, ethyl, n-propyl, isopropyl, n-butyl, isobutyl, tert-butyl, trifluoromethyl, methoxy, ethoxy, n-propoxy, isopropoxy, n-butoxy, tert-butoxy, sec-butoxy, n-pentyloxy, n-hexyloxy, methylthio, ethylthio, n-propylthio, isopropylthio, n-butylthio, tert-butylthio, sec-butylthio, n-pentylthio, n-hexylthio, -NH(CO)CH₃, F, Cl, Br or cyano.

In some embodiments, the compound represented by general Formula (I) has the structure represented by Formula (I-1), and the compound has the structure represented by Formula 1-1: 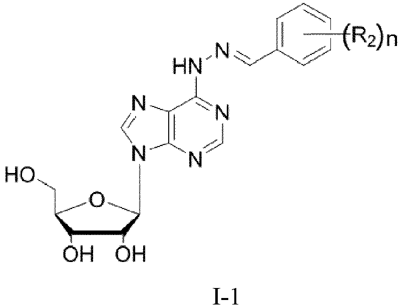 R₂ represents a substituent attached to the benzene ring;
n is 1, 2, 3, 4 or 5;
Each R₂ is independently selected from the group consisting of phenyl, halophenyl, amino-substituted phenyl, benzyloxy, halobenzyloxy, phenylamino, heteroaryl, cycloalkyl, heterocycloalkyl, C₁₋₆ alkyl, C₁₋₆ heteroalkyl, C₁₋₆ haloalkyl, C₂₋₁₀ alkenyl (such as C₂₋₆ alkenyl), C₁₋₆ alkoxy, C₁₋₆ haloalkoxy, C₁₋₆ alkylamino, acylamino, halogen, hydroxy, cyano or -NHC(O)R¹⁰, wherein R¹⁰ is C₁₋₄ alkyl.

In some embodiments, each R₂ is independently selected from the group consisting of C₁₋₆ alkyl, C₁₋₆ heteroalkyl, C₁₋₆ haloalkyl, C₁₋₆ alkoxy, C₁₋₆ haloalkoxy, C₁₋₆ alkylamino, acylamino, phenyl, benzyloxy, halobenzyloxy, phenylamino, 5- to 6-membered heterocycloalkyl, - NH(CO)CH₃, halogen, hydroxy, or cyano.

In some embodiments, each R₂ is independently selected from the group consisting of phenyl, halophenyl, amino-substituted phenyl, benzyloxy, halobenzyloxy, phenylamino, heteroaryl, cycloalkyl, heterocycloalkyl, C₁₋₆ alkyl, C₁₋₆ haloalkyl, C₂₋₁₀ alkenyl (such as C₂₋₆ alkenyl), C₁₋₆ alkoxy, -NHC(O)R¹⁰, halogen or cyano, wherein R¹⁰ is C₁₋₄ alkyl.

In some embodiments, each R₂ is independently selected from the group consisting of methyl, trifluoromethyl, ethyl, n-propyl, isopropyl, n-butyl, isobutyl, tert-butyl, C₁₋₃ alkoxy, phenyl, diphenylamino, benzyloxy, halobenzyloxy, pyridin-2-yl, 1H-imidazol-1-yl, pyrrolidin-1-yl, - NH(CO)CH₃, F, Cl, Br or cyano.

In some embodiments, each R₂ is independently selected from the group consisting of - NH(CO)(R'), benzyloxy, halobenzyloxy, trifluoromethyl, pyridin-2-yl, phenyl, pyrrolidin-1-yl, 1H-imidazol-1-yl, C₁₋₃ alkoxy, diphenylamino. Each R' is independently selected from the group consisting of phenyl, halophenyl, amino-substituted phenyl, benzyloxy, halobenzyloxy, phenylamino, heteroaryl, cycloalkyl, heterocycloalkyl, C₁₋₆ alkyl, halogenated C₁₋₆ alkyl, C₂₋₁₀ alkenyl (such as C₂₋₆ alkenyl), C₁₋₆ alkoxy, C₁₋₆ alkylthio, -NHC(O)R¹⁰, halogen or cyano, wherein R¹⁰ is C₁₋₆ alkyl.

In some embodiments, R' is C₁₋₆ alkyl, such as C₁₋₃ alkyl, such as methyl.

In some embodiments, each R₂ is independently selected from halobenzyloxy.

In some embodiments, each R₂ is independently selected from 4-fluorobenzyloxy.

In some embodiments, n=1.

In some embodiments, n=2.

In some embodiments, n=3.

In some embodiments, n=4.

In some embodiments, n=5.

In some embodiments, each R₂ is independently selected from benzyloxy or halobenzyloxy.

In some embodiments, R₂ is halobenzyloxy, such as chlorobenzyloxy.

In some embodiments, the compound represented by general Formula (I) has a structure represented by Formula (1-2), and the compound has a structure represented by Formula I-2:

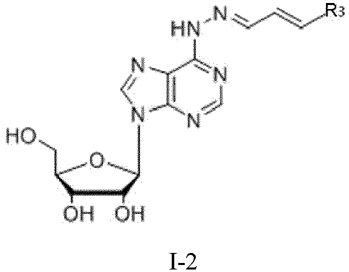

R₃ is selected from the group consisting of phenyl, halophenyl, amino-substituted phenyl, C₁₋₄ alkylamino-substituted phenyl, di(C₁₋₄ alkyl)amino-substituted phenyl, C₁₋₈ alkyl or C₂₋₈ alkenyl.

In some embodiments, R₃ is dimethylamino-substituted phenyl.

In some embodiments, R₃ is 1-heptenyl.

The second aspect of the present disclosure provides a method for preparing the compound of general Formula (I), or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, comprising: 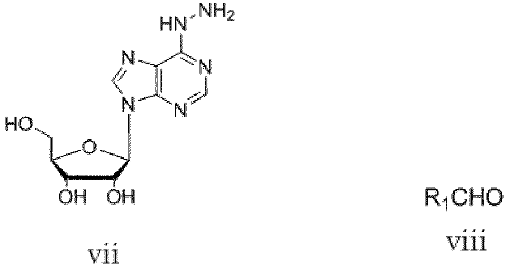 reacting a compound of Formula (vii) with a substituted formaldehyde represented by Formula (viii) to obtain the compound represented by general Formula (I), wherein the definition of R₁ is the same as that described in the first aspect of the present disclosure.

In some embodiments, the compound of Formula (vii) reacts with a substituted formaldehyde (viii) in a methanol solution under microwaves at 70°C to 90°C;

In some embodiments, the compound of Formula (vii) is prepared by hydrazinolyzing a compound of Formula (vi) with hydrazine hydrate (N₂H₄·H₂O) at 60~80°C

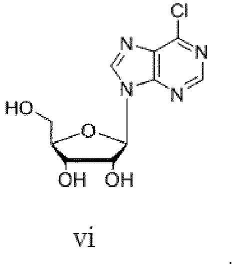

In some embodiments, the method of synthesizing the compound of Formula (I) is as follows: 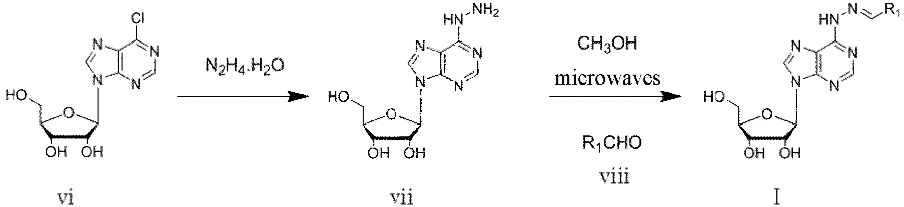 wherein, the compound of Formula (vi) is the starting material and reacts with hydrazine hydrate at 60~80°C to produce the compound of Formula (vii); then the compound of Formula (vii) is reacted with a substituted formaldehyde (viii) in methanol solution at 70~90°C under microwaves to obtain the 6-hydrazinoadenosine compound (I), wherein the substitution of R₁ is the same as that described in the first aspect of the present disclosure, and is selected as required.

The third aspect of the present disclosure provides a pharmaceutical composition, which comprises at least one of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, and one or more pharmaceutically acceptable carriers or excipients.

In some embodiments, the above-mentioned pharmaceutical composition further comprises: a drug for crossing the blood-brain barrier, which is selected from the group consisting of a drug for treating a disease or disorder of the central nervous system, a neurotoxin antidote, and a drug for treating a brain glioma.

The fourth aspect of the present disclosure provides use of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, or the pharmaceutical composition as described in the third aspect of the present disclosure in the manufacture of a medicament as an A_{2A} adenosine receptor agonist, or
use in the manufacture of a medicament for the prevention and/or treatment of a human pathological condition or symptom, wherein the prevention or treatment of a human pathological condition or symptom is related to the activity of A_{2A} adenosine receptor, and the prevention and/or treatment of a human pathological condition or symptom requires agonizing of the A_{2A} adenosine receptor.

In some embodiments, the human pathological condition or symptom is selected from the following: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, founder's disease, glaucoma, ocular hypertension, spinal cord injury, myocardial infarction, and acute myocardial infarction.

The fifth aspect of the present disclosure provides use of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, or the pharmaceutical composition as described in the third aspect of the present disclosure in the manufacture of a medicament for diagnosing a human myocardial perfusion abnormality.

The sixth aspect of the present disclosure provides use of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, or the pharmaceutical composition as described in the third aspect of the present disclosure in the manufacture of a medicament for increasing a blood-brain barrier permeability of a subject receiving a therapeutic drug, wherein the subject is benefited from the increased blood-brain barrier permeability for delivering the therapeutic drug across the blood-brain barrier.

In some embodiments, the therapeutic drug is selected from the following: a drug that is effective in treating a disease or disorder of the central nervous system, a neurotoxin antidote, and a drug for treating a brain glioma.

The seventh aspect of the present disclosure provides a pharmaceutical composition, which comprises:
at least one of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, and
a drug for crossing the blood-brain barrier, which is selected from the group consisting of a drug for treating a disease or disorder of the central nervous system, a neurotoxin antidote, and a drug for treating a brain glioma, and
one or more pharmaceutically acceptable carriers or excipients.

The eighth aspect of the present disclosure provides a method for prevention and/or treatment of a human pathological condition or symptom, comprising administering to a patient in need of such treatment a therapeutically effective amount of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, or the pharmaceutical composition according to the third aspect of the present disclosure, wherein the human's pathological condition or symptom is related to the activity of A_{2A} adenosine receptor, and the prevention or treatment of the pathological condition or symptom of the patient requires agonizing of the A_{2A} adenosine receptor.

The ninth aspect of the present disclosure provides the compound represented by the general Formula (I), or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, for use in prevention and/or treatment of a human pathological condition or symptom, wherein the human pathological condition or symptom is related to the activity of A_{2A} adenosine receptor, and the prevention or treatment of the human pathological condition or symptom requires agonizing of the A_{2A} adenosine receptor.

The tenth aspect of the present disclosure provides the compound represented by the general Formula (I), or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure,
for use in agonizing A_{2A} adenosine receptor or vasodilating a coronary artery, or
for use in diagnosing a human myocardial perfusion abnormality, or
for use in increasing a blood-brain barrier permeability of a subject receiving a therapeutic drug, in which the subject benefits from the increased blood-brain barrier permeability for delivering the therapeutic drug across the blood-brain barrier,
preferably, the therapeutic drug is selected from: a drug for treating a disease or disorder of the central nervous system, a neurotoxin antidote, and a drug for treating a brain glioma.

The eleventh aspect of the present disclosure also provides a method for diagnosing a human myocardial perfusion abnormality, comprising administering to a patient in need of such diagnosis a diagnostically effective amount of the compound represented by the general Formula (I), or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, or the pharmaceutical composition as described in the third aspect of the present disclosure.

The twelfth aspect of the present disclosure also provides a method for increasing the permeability of the blood-brain barrier of a subject receiving a therapeutic drug, the method comprising administering to the subject an effective amount of the compound represented by the general Formula (I), or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, or the pharmaceutical composition as described in the third aspect of the present disclosure, wherein the subject benefits from the increased permeability of the blood-brain barrier for delivering the therapeutic drug across the blood-brain barrier.

According to some embodiments of the present disclosure, in the method described in the twelfth aspect of the present disclosure, the therapeutic drug is selected from: a drug for treating a disease or disorder of the central nervous system, a neurotoxin antidote, and a drug for treating a brain glioma.

According to some embodiments of the present disclosure, the human pathological condition or symptom described in the present disclosure is selected from: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, founder's disease, glaucoma, ocular hypertension, spinal cord injury, myocardial infarction, and acute myocardial infarction.

In some embodiments, the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, as described in the first aspect of the present disclosure, or the pharmaceutical composition as described in the third aspect of the present disclosure, has one or more of the following beneficial effects:
- minor nerve damage;
- being able to deliver a drug with a larger molecular weight;
- having good pharmacokinetic characteristics.

Description of terms:
As used in this application, the term "alkyl" used alone or in combination with other terms refers to a saturated linear or branched monovalent hydrocarbon group, preferably having 1-6, 1-4 or 1-3 carbon atoms. For example, "C₁₋₆ alkyl" refers to a saturated linear or branched monovalent hydrocarbon group having 1 to 6 carbon atoms. Typical examples of "alkyl" include, but are not limited to, methyl, ethyl, n-propyl, isopropyl, n-butyl, isobutyl, tert-butyl, n-pentyl, tert-pentyl, neopentyl, hexyl, etc.

The term "hydroxyl" as used herein refers to -OH.

The term "halogen" as used herein refers to fluorine, chlorine, bromine or iodine. Preferred halogen is fluorine, chlorine or bromine.

The term "halo" as used herein refers to substitution by one or more halogen atoms.

The term "halogenated C₁₋₆ alkyl" as used herein refers to a C₁₋₆ alkyl mono- or polysubstituted by halogen such as fluorine, chlorine, bromine or iodine. Preferred haloalkyl is chloromethyl, chloroethyl, dichloroethyl, trifluoromethyl, difluoromethyl, monofluoromethyl, and the like.

The term "C₁₋₆ alkylamino" as used herein refers to an amino group substituted with one C₁₋₆ alkyl. Typical examples of "C₁₋₆ alkylamino" include but are not limited to methylamino, ethylamino, propylamino, butylamino and so on.

As used in this application, the term "aryl" used alone or in combination with other terms refers to monocyclic or polycyclic (for example, having 2, 3 or 4 condensed rings) aromatic hydrocarbon group, such as but not limited to, phenyl, 1-naphthyl, 2-naphthyl, anthryl, phenanthryl, etc. In certain embodiments, the aryl is a C₆₋₁₄ aryl. In certain embodiments, the aryl is a C₆₋₁₀ aryl. In certain embodiments, the aryl is a naphthyl ring or phenyl ring. In certain embodiments, the aryl is phenyl.

As used in this application, the term "heteroaryl" used alone or in combination with other terms refers to a monocyclic or polycyclic (e.g., having 2, 3 or 4 condensed rings) aromatic heterocyclic moiety having one or more heteroatom ring members selected from nitrogen, sulfur and oxygen. In certain embodiments, the heteroaryl has 1, 2, 3 or 4 heteroatom ring members. In certain embodiments, the heteroaryl has 1, 2 or 3 heteroatom ring members. In certain embodiments, the heteroaryl has 1 or 2 heteroatom ring members. In certain embodiments, the heteroaryl has 1 heteroatom ring member. In certain embodiments, the heteroaryl is 5- to 10-membered or 5- to 6-membered. In certain embodiments, the heteroaryl is 5-membered. In certain embodiments, the heteroaryl is 6-membered. Examples of the heteroaryl include, but are not limited to, pyrrolyl, imidazolyl, thiazolyl, furyl or pyridyl, etc.

The term "cycloalkyl" as used herein refers to a saturated cyclic hydrocarbon group having 3 to 12 carbon atoms and having a monocyclic or bicyclic or multiple fused rings (including fused and bridged ring systems), preferably having 3-10, 3-8, 5-8, 3-6 or 5-6 carbon atoms. Typical examples of "cycloalkyl" include, but are not limited to, monocyclic structures, such as cyclopropyl, cyclobutyl, cyclopentyl, cyclohexyl, cycloheptyl, cyclooctyl, etc.; bicyclic structures, such as bicyclo[2.2.1]heptyl, and polycyclic structures such as adamantyl and the like.

The term "heterocycloalkyl" as used herein refers to a cycloalkyl as defined herein that contains one, two or more heteroatoms independently selected from N, O and S. Typical examples of "heterocycloalkyl" include, but are not limited to, tetrahydrofuranyl, tetrahydrothienyl, pyrrolidinyl, piperazinyl, thiazinyl, piperidinyl, morpholinyl and the like.

As used in this application, the term "aralkyl" used alone or in combination with other terms refers to a lower alkyl or cycloalkyl as defined above, in which one hydrogen atom has been substituted by an aryl as defined above, or in the case of cycloalkyl, two adjacent carbon atoms are fused in benzo form to a substituted or unsubstituted phenyl to form a bicyclic group.

As used in this application, the term "heteroalkyl" used alone or in combination with other terms refers to an alkyl in which one or more carbon atoms are substituted by heteroatoms independently selected from S, O and N.

The term "C₁₋₆ alkoxy" as used herein refers to -OR¹¹, where R¹¹ is a C₁₋₆ alkyl as defined herein. Typical examples of "C₁₋₆ alkoxy" include, but are not limited to, methoxy, ethoxy, n-propoxy, isopropoxy, n-butoxy, tert-butoxy, sec-butoxy, n-pentyloxy, n-hexyloxy, 1,2-dimethylbutoxy, etc.

The term "C₁₋₆ alkylthio" as used herein refers to -SR¹¹, where R¹¹ is a C₁₋₆ alkyl as defined herein. Typical examples of "C₁₋₆ alkylthio" include, but are not limited to, methylthio, ethylthio, n-propylthio, isopropylthio, n-butylthio, tert-butylthio, sec-butylthio, n-pentylthio, n-hexylthio, 1,2-dimethylbutylthio, etc.

When the names of compounds used herein is inconsistent with the chemical structural formulae, the chemical structural Formulae shall prevail.

According to some embodiments of the present invention, the pharmaceutically acceptable salt of the compound of general Formula (I) described in this application includes its inorganic or organic acid salt, and inorganic or organic base salt, and this application relates to all forms of the above-mentioned salt, which includes but not limited to: sodium salt, potassium salt, calcium salt, lithium salt, meglumine salt, hydrochloride, hydrobromide, hydroiodide, nitrate, sulfate, hydrogensulfate, phosphate, hydrogenphosphate, acetate, propionate, butyrate, oxalate, trimethylacetate, adipate, alginate, lactate, citrate, tartrate, succinate, maleate, fumarate, picrate, aspartate, gluconate, benzoate, methanesulfonate, ethanesulfonate, benzenesulfonate, p-toluenesulfonate and pamoate, etc.

According to some embodiments of the present invention, the compound of general Formula (I) of the present invention can form a pharmaceutically acceptable ester with an organic or inorganic acid, and the pharmaceutically acceptable ester includes phosphate, sulfate, nitrate, formate, acetate, propionate, butyrate, valerate, and caproate, etc., that are hydrolyzable in vivo.

The carrier of the present invention includes, but is not limited to: ion exchanger, alumina, aluminum stearate, lecithin, serum protein such as human albumin, buffer substance such as phosphate, glycerol, sorbic acid, potassium sorbate, partial glyceride mixture of plant saturated fatty acid, water, salt or electrolyte, such as protamine sulfate, disodium hydrogen phosphate, potassium hydrogen phosphate, sodium chloride, zinc salt, colloidal silica, magnesium trisilicate, polyvinylpyrrolidone, cellulosic substance, polyethylene glycol, sodium carboxymethylcellulose, polyacrylate, beeswax, lanolin.

The term "excipient" as used in the present invention refers to an additive other than the main drug in a pharmaceutical preparation. It is stable in nature, has no incompatibility with the main drug, does not produce side effects, does not affect therapeutic effect, is not easy to be deformed, dried, cracked, funked, wormed, is harmless to the human body, has no physiological effect, and does not produce chemical or physical effect with the main drug, does not affect the content determination of the main drug, etc. For example, the binder, filler, disintegrant, lubricant in tablets; the preservative, antioxidant, flavor, fragrance, cosolvent, emulsifier, solubilizer, osmotic pressure regulator, coloring agent, etc. in oral liquid preparations can all be called excipients.

The pharmaceutical composition described in this application can be administered through various routes, such as oral tablet, capsule, powder, oral liquid, injection and transdermal preparation. The above-mentioned various dosage forms of drugs can be prepared according to conventional methods in the field of pharmacy. According to conventional pharmaceutical practices, pharmaceutically acceptable carriers include diluent, filler, disintegrant, wetting agent, lubricant, coloring agent, flavoring agent or other conventional additives. Typical pharmaceutically acceptable carriers include, for example, microcrystalline cellulose, starch, crospovidone, povidone, polyvinylpyrrolidone, maltitol, citric acid, sodium laurylsulfonate or magnesium stearate, etc..

According to the present application, the pharmaceutical composition can be administered in any of the following ways: oral, spray inhalation, rectal administration, nasal administration, buccal administration, vaginal administration, topical administration, parenteral administration such as subcutaneous, intravenous, intramuscular, intraperitoneal, intrathecal, intraventricular, intrasternal and intracranial injection or infusion, or administration with the aid of an explanted reservoir.

As stated in this article, "effective amount" refers to an amount that is sufficient to treat or prevent or diagnose a disease of a patient, but is low enough to avoid serious side effects (at a reasonable benefit/risk ratio) within the scope of reasonable medical judgment. The therapeutically or prophylactically or diagnostically effective amount of the compound will vary according to the specifically selected compound (for example, considering the potency, effectiveness and half-life of the compound), the selected route of administration, the disease to be treated or prevented or diagnosed, the severity of the disease to be treated or prevented or diagnosed, the age, size, weight and physical disease of the patient being treated, the medical history of the patient being treated, the duration of treatment or prevention or diagnosis, the nature of concurrent therapy, the required treatment or prevention or diagnosis effect, etc., but it could still be routinely determined by those skilled in the art.

In addition, it should be pointed out that the specific dosage and usage of the compound of general Formula (I) described in this application for different patients are determined by many factors, including the age, weight, gender, natural health status, nutritional status of the patient, the activity strength, administration time, metabolic rate of the compound, the severity of the disease and the subjective judgment of the physician. The preferred dosage here is between 0.001 to 100 mg/kg body weight/day.

### Specific Models for Carrying Out the Invention

The present disclosure can be further described through the following examples and test examples. However, the scope of the present disclosure is not limited to the following examples or test examples. Those skilled in the art can understand that various changes and modifications can be made to the present disclosure without departing from the spirit and scope of the present disclosure. This disclosure provides a general and/or specific description of the materials and methods used herein. Although many materials and operating methods used to achieve the purpose of the present disclosure are well-known in the art, the present disclosure is still described herein as much detail as possible.

For all the following examples, standard operations and purification methods known to those skilled in the art can be used. Unless otherwise stated, all temperatures were expressed in °C (Celsius). The structure of compound was determined by nuclear magnetic resonance (NMR) or mass spectrometry (MS). The melting point m.p. of compound was determined by RY-1 melting point instrument. The thermometer had not been corrected. The m.p. was given in °C. ¹H NMR was measured by JNM-ECA-400 nuclear magnetic resonance instrument of JEOL. The mass spectrum was measured by API3000 (ESI) instrument. All reaction solvents that were not specified were subject to standardized pretreatment.

### Example 1: Synthesis of (2R,3R,4S,5R)-2-{6-{ 2-[(E)-(1H-pyrrol-2-yl)methylene] hydrazino}-9H-purin-9-yl}-5-(hydroxymethyl)tetrahydrofuran-3,4-diol (Compound 1)

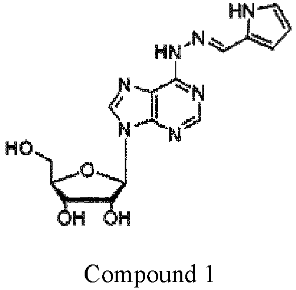

### 1.1 Synthesis of (2R,3R,4S,5R)-2-(6-hydrazino-9H-purin-9-yl)-5-(hydroxymethyl) tetrahydrofuran-3,4-diol (II)

To 10ml of hydrazine hydrate (65wt% aqueous solution), 5g (0.018mol) of (2R,3R,4S,5R)-2-(6-chloro-9H-purin-9-yl)-5-(hydroxymethyl)tetrahydrofuran-3,4-diol (vi) was added, heated to 70°C while stirring, continued heating for 2 hours until the reactant (I) disappeared, the reaction progress was monitored by TLC (CH₂Cl₂:MeOH = 3:1 (v/v)). Then, the reaction mixture was heated to 25°C, diluted with 2-propanol (50ml) and stirred overnight. The separated precipitate was filtered to obtain 4.8 g of (2R,3R,4S,5R)-2-(6-hydrazino- 9H-purin-9-yl)-5-(hydroxymethyl)tetrahydrofuran-3,4-diol (vii) as white solid, which was directly used in the next reaction.

### 1.2 Synthesis of (2R,3R,4S,5R)-2-{6-{2-[(E)-(1H-pyrrol-2-yl)methylene]hydrazino}- 9H-purin-9-yl}-5-(hydroxymethyl)tetrahydrofuran-3,4-diol (Compound 1)

0.5g (0.0018mol) of (2R,3R,4S,5R)-2-(6-hydrazino-9H-purin-9-yl)-5-(hydroxymethyl) tetrahydrofuran-3,4-diol (II) and 0.19g (0.002mol) of pyrrole-2-carbaldehyde (1H-pyrrole-2-carbaldehyde, 1.1 equivalent) were mixed in methanol (20ml) and heated by microwave at 70°C for 30 minutes.

The crude product was precipitated from methanol. After filtration, the crude product was further purified by medium pressure preparative chromatography using C18 reverse phase column, and 323 mg of white solid (Compound 1) was obtained. m.p. 160°C; ¹H NMR (DMSO-d₆): δ (ppm) 11.52(s, 1H), 11.44(s, 1H), 8.50(s, 1H), 8.34(s, 1H), 8.28(s, 1H), 6.90(s, 1H), 6.43(s, 1H), 6.14(s, 1H), 5.95(d, 1H, J=6.0Hz), 5.54(d, 1H, J=6.0Hz), 5.40(dd, 1H, J=2.0Hz, 4.8Hz), 5.26(d, 1H, J=4.4Hz), 4.65(dd, 1H, J=5.6Hz, 5.6Hz), 4.18(d, 1H, J=3.6Hz), 3.99(d, 1H, J=2.8Hz), 3.73-3.55(m, 2H); HRMS (ESI+) m/z [M + H]⁺ calculated for C₁₅H₁₇N₇O₄: 360.1415; found: 360.1415.

The following compounds could be prepared by referring to the method of Example 1, using different reactants (such as the above-mentioned compound of Formula viii, various substituted formaldehydes) in place of pyrrole-2-carboxaldehyde in step 1.2.

### Example 2: Synthesis of (2R,3S,4R,5R)-2-(hydroxymethyl)-5-{6-{2-[(E)-3-(methylthio) propylene]hydrazino}-9H-purin-9-yl}tetrahydrofuran-3,4-diol (Compound 2)

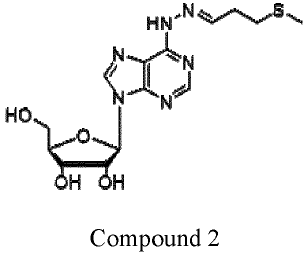

The method of step 1.2 in Example 1 was adopted, in which 3-(methylsulfanyl)propanal was used in place of pyrrole-2-carboxaldehyde to prepare Compound 2, and 424 mg of white solid (Compound 2) was obtained. m.p. 94°C; ¹H NMR (DMSO-d₆): δ (ppm) 11.42(s, 1H), 8.47(s, 1H), 8.31(s, 1H), 7.72(t, 1H, J=5.2Hz), 5.93(d, 1H, J=6.0Hz), 5.5-(d, 1H, J=6.0Hz), 5.34(t, 1H, J=6.0Hz), 5.23(d, 1H, J=4.8Hz), 4.61(dd, 1H, J=5.2Hz, 6.0Hz), 4.16(d, 1H, J=3.6Hz), 3.97(d, 1H, J=3.6Hz), 3.71-3.54(m, 2H), 2.71(t, 2H, J=7.2Hz), 2.58(t, 2H, J=6.2Hz), 2.10(s, 3H); HRMS (ESI+) m/z [M + H]⁺ calculated for C₁₄H₂₀N₆O₄S: 369.1340; found: 369.1340.

### Example 3: Synthesis of N-{4-{(E)-{2-{9-[(2R,3R,4S,5R)-3,4-dihydroxy-5-(hydroxymethyl) tetrahydrofuran-2-yl]-9H-purin-6-yl}hydrazino }methyl}phenyl}acetamide (Compound 3)

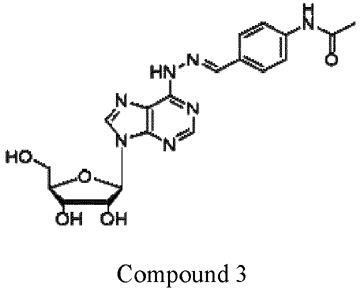

The method of step 1.2 in Example 1 was adopted, and 4-acetamidobenzaldehyde (N-(4-formylphenyl)acetamide) was used in place of pyrrole-2-carbaldehyde to prepare Compound 3, and 331 mg of white solid (Compound 3) was obtained.

m.p. 170°C; ¹H NMR (DMSO-d₆): δ (ppm) 11.72(s, 1H), 10.13(s, 1H), 8.53(s, 1H), 8.38(s, 1H), 7.67(s, 4H), 5.96(d, 1H, J=5.6Hz), 5.51(d, 1H, J=6.0Hz), 5.33(t, 1H, J=5.6Hz), 5.24(d, 1H, J=4.8Hz), 4.63(d, 1H, J=5.6Hz), 4.17(s, 1H), 3.98(s, 1H), 3.72-3.55(m, 2H), 2.07(s, 3H); HRMS (ESI+) m/z [M + H]⁺ calculated for C₁₉H₂₁N₇O₅: 428.1677; found: 428.1677.

### Example 4: Synthesis of (2R,3R,4S,5R)-2-{6-{2-[(E)-3,4-bis(benzyloxy)benzylidene] hydrazino}-9H-purin-9-yl}-5-(hydroxymethyl)tetrahydrofuran-3,4-diol (Compound 4)

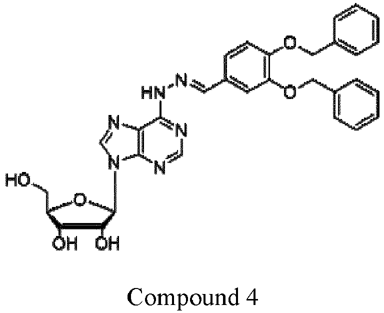

The method of step 1.2 in Example 1 was adopted, in which 3,4-bis(benzyloxy)benzaldehyde was used in place of pyrrole-2-carbaldehyde to prepare Compound 4, and 880 mg of white solid (Compound 4) was obtained. m.p. 186°C; ¹H NMR (DMSO-d₆): 8 (ppm) 11.71(s, 1H), 8.54(s, 1H), 8.39(s, 1H), 8.27(s, 1H), 7.53-7.14(m, 13H), 5.95(s, 1H), 5.51(s, 1H), 5.34(s, 1H), 5.25(s, 1H), 5.21(s, 4H), 4.63(s, 1H), 4.17(s, 1H), 3.99(s, 1H), 3.72-3.58(m, 2H); HRMS (ESI+) m/z [M + H]⁺ calculated for C₃₁H₃₀N₆O₆: 583.2300; found: 583.2298.

### Example 5: Synthesis of (2R,3R,4S,5R)-2-{6-{2-{(E)-[5-(4-bromophenyl)furan-2-yl]methylene} hydrazino}-9H-purin-9-yl}-5-(hydroxymethyl)tetrahydrofuran-3,4-diol (Compound 5)

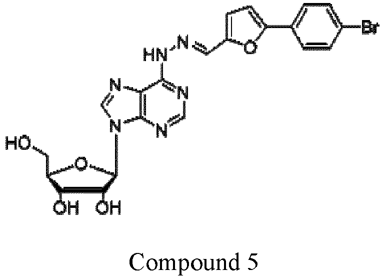

The method of step 1.2 in Example 1 was adopted, in which 5-(4-bromophenyl)furan-2-carbaldehyde was used in place of pyrrole-2-carbaldehyde to prepare Compound 5, and 712 mg of yellow solid (Compound 5) was obtained. m.p. 162°C; ¹H NMR (DMSO-d₆): δ (ppm) 11.99(br, 1H), 8.58(s, 1H), 8.44(s, 1H), 8.35(s, 1H), 7.76(d, 2H, J=8.4Hz), 7.68(d, 2H, J=8.4Hz), 7.22(d, 1H, J=3.6Hz), 7.02(d, 1H, J=3.6Hz), 5.98(d, 1H, J=6.0Hz), 5.55-5.28(br, 3H), 4.65(s, 1H), 4.19(s, 1H), 4.01(s, 1H), 3.73-3.57(m, 2H); HRMS (ESI+) m/z [M + H]⁺ calculated for C₂₁H₁₉BrN₆O₅: 515.0673; found: 515.0673.

### Example 6: Synthesis of (2R,3R,4S,SR)-2-{6-{2-[(E)-2,4-bis(trifluoromethyl)benzylidene] hydrazino}-9H-purin- 9-yl}-5-(hydroxymethyl)tetrahydrofuran-3,4-diol (Compound 6)

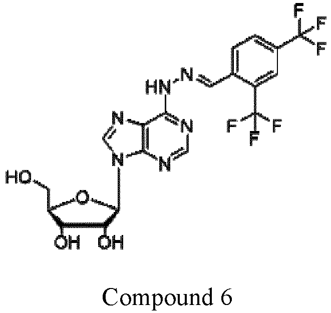

The method of step 1.2 in Example 1 was adopted, in which 2,4-bis(trifluoromethyl)benzaldehyde was used in place of pyrrole-2-carbaldehyde to prepare Compound 6, and 592 mg of white solid (Compound 6) was obtained. m.p. 200°C; ¹H NMR (DMSO-d₆): δ (ppm) 12.42(s, 1H), 8.78(s, 1H), 8.65(s, 1H), 8.62(d, 1H, J=8.4Hz), 8.50(s, 1H), 8.19(d, 1H, J=8.4Hz), 8.08(s, 1H), 6.01(d, 1H, J=6.0Hz), 5.57(d, 1H, J=6.0Hz), 5.31-5.28(m, 2H), 4.65(dd, 1H, J=4.8Hz, 6.0Hz), 4.20(d, 1H, J=3.6Hz), 4.01(d, 1H, J=3.2Hz), 3.75-3.57(m, 2H); HRMS (ESI+) m/z [M + H]⁺ calculated for C₁₉H₁₆F₆N₆O₄: 507.1210; found: 507.1209.

### Example 7: Synthesis of (2R,3R,4S,5R)-2-{6-{2-{(E)-4-[(4-fluorobenzyl)oxy]benzylidene} hydrazino}-9H-purin-9-yl}-5-(hydroxymethyl)tetrahydrofuran-3,4-diol (Compound 7)

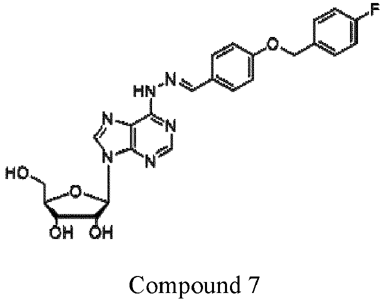

The method of step 1.2 in Example 1 was adopted, in which 4-(4-fluorobenzyloxy)benzaldehyde (4-[(4-fluorophenyl)methoxy]benzaldehyde) was used in place of pyrrole-2-carboxaldehyde to prepare Compound 7, and 859 mg of white Solid (Compound 7) was obtained. m.p. 206°C; ¹H NMR (DMSO-d₆): δ (ppm) 11.69(s, 1H), 8.54(s, 1H), 8.38(s, 1H), 8.31(s, 1H), 7.70(d, 2H, J=8.8Hz), 7.53(t, 2H, J=5.6Hz), 7.24(t, 2H, J=8.8Hz), 7.11(d, 2H, J=8.4Hz), 5.97(d, 1H, J=6.0Hz), 5.52(d, 1H,J=6.4Hz), 5.34(t, 1H, J=5.20Hz), 5.24(d, 1H, J=4.4Hz), 5.14(s, 2H), 4.63(d, 1H, J=5.6Hz), 4.18(s, 1H), 3.99(s, 1H), 3.72-3.56(m, 2H); HRMS (ESI+) m/z [M + H]⁺ calculated for C₂₄H₂₃FN₆O₅: 495.1787; found: 495.1787.

### Example 8: Synthesis of (2R,3R,4S,5R)-2-{6-{2-[(E)-3-(benzyloxy)benzylidene]hydrazino} -9H-purin-9-yl}-5-(hydroxymethyl)tetrahydrofuran-3,4-diol (Compound 8)

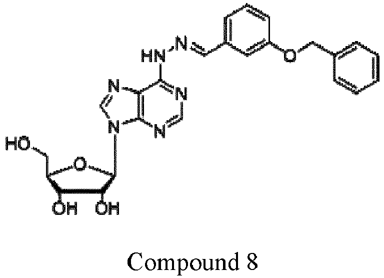

The method of step 1.2 in Example 1 was adopted, in which 3-(benzyloxy)benzaldehyde was used in place of pyrrole-2-carbaldehyde to prepare Compound 8, and 568 mg of white solid (Compound 8) was obtained. m.p. 140°C; ¹H NMR (DMSO-d₆): δ (ppm) 11.80(s, 1H), 8.57(s, 1H), 8.40(s, 1H), 8.32(s, 1H), 7.51-7.32(m, 8H), 7.07-7.04(m, 1H), 5.97(d, 1H, J=5.6Hz), 5.49(d, 1H,J=6.4Hz), 5.29(t, 1H, J=5.20Hz), 5.21(d, 1H, J=4.8Hz), 5.17(s, 2H), 4.63(dd, 1H, J=5.6Hz, 5.6Hz), 4.18(dd, 1H, J=3.6Hz, 4.8Hz), 3.99(d, 1H, J=3.2Hz), 3.73-3.55(m, 2H); HRMS (ESI+) m/z [M + H]⁺ calculated for C₂₄H₂₄N₆O₅: 477.1881; found: 477.1883.

### Example 9: Synthesis of (2R,3S,4R,5R)-2-(hydroxymethyl)-5-{6-{2-[(E)-4-(pyridin-2-yl) benzylidene]hydrazino}-9H-purin-9-yl}tetrahydrofuran-3,4-diol (Compound 9)

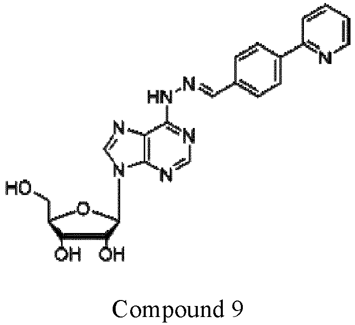

The method in step 1.2 of Example 1 was adopted, in which 4-(pyridin-2-yl)benzaldehyde was used in place of pyrrole-2-carbaldehyde to prepare Compound 9, and 652 mg of white solid (Compound 9) was obtained. m.p. 236°C; ¹H NMR (DMSO-d₆): δ (ppm) 11.88(s, 1H), 8.70(d, 1H, J=5.6Hz), 8.58(s, 1H), 8.43(s, 2H), 8.20(d, 2H, J=8.4Hz), 8.03(d, 1H,J=8.0Hz), 7.92(d, 1H, J=9.2Hz), 7.88(d, 2H, J=8.4Hz), 7.40-7.37(m, 1H), 5.99(d, 1H, J=6.0Hz), 5.51(d, 1H,J=6.4Hz), 5.30(t, 1H, J=6.4Hz), 5.22(d, 1H, J=4.8Hz), 4.64(dd, 1H, J=5.6Hz, 5.6Hz), 4.19(dd, 1H, J=3.6Hz, 4.4Hz), 4.00(d, 1H, J=3.2Hz), 3.73-3.57(m, 2H); HRMS (ESI+) m/z [M + H]⁺ calculated for C₂₂H₂₁N₇O₄: 448.1728; found: 448.1729.

### Example 10: Synthesis of (2R,3R,4S,5R)-2-{6-{2-[(E)-[1,1'-biphenyl]-4-ylmethylene] hydrazino}-9H-purin-9-yl}-5-(hydroxymethyl)tetrahydrofuran-3,4-diol (Compound 10)

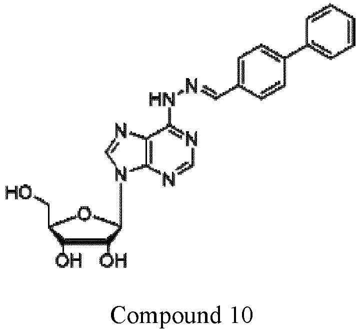

The method of step 1.2 in Example 1 was adopted, in which 4-phenylbenzaldehyde was used in place of pyrrole-2-carboxaldehyde to prepare Compound 10, and 698 mg of white solid (Compound 10) was obtained. m.p. 170°C; ¹H NMR (DMSO-d₆): δ (ppm) 11.87(s, 1H), 8.59(s, 1H), 8.43(s, 2H), 7.86 (d, 2H, J=8.0Hz), 7.78(d, 2H, J=8.4Hz), 7.74(d, 2H, J=7.2Hz), 7.51(t, 2H, J=7.2Hz), 7.41(t, 1H, J=7.6Hz), 5.99(d, 1H, J=5.6Hz), 5.54(d, 1H,J=6.4Hz), 5.34(t, 1H, J=5.6Hz), 5.26(d, 1H, J=4.8Hz), 4.64(dd, 1H, J=5.2Hz, 5.6Hz), 4.19(dd, 1H, J=3.6Hz, 4.4Hz), 4.00(d, 1H, J=4.0Hz), 3.74-3.57(m, 2H); HRMS (ESI+) m/z [M + H]⁺ calculated for C₂₃H₂₂N₆O₄: 447.1775; found: 447.1775.

### Example 11: Synthesis of (2R,3S,4R,5R)-2-(hydroxymethyl)-5-{6-{2-[(E)-4-(pyrrolidin-1-yl) benzylidene]hydrazino}-9H-purin-9-yl}tetrahydrofuran-3,4-diol (Compound 11)

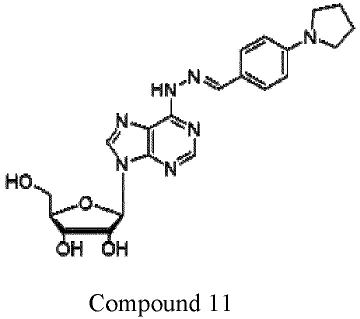

The method of step 1.2 in Example 1 was adopted, in which 4-(1-pyrrolidin-1-yl)benzaldehyde was used in place of pyrrolidin-2-carbaldehyde to prepare Compound 11, and 664 mg of white solid (Compound 11) was obtained. m.p. 202°C; ¹H NMR (DMSO-d₆): δ (ppm) 11.43(s, 1H), 8.48(s, 1H), 8.33(s, 1H), 8.24(s, 1H), 7.54 (d, 2H, J=8.4Hz), 6.59(d, 2H, J=8.4Hz), 5.95(d, 1H, J=6.4Hz), 5.48(d, 1H,J=6.0Hz), 5.36(t, 1H, J=4.8Hz), 5.20(d, 1H, J=4.8Hz), 4.63(d, 1H, J=5.2Hz), 4.17(d, 1H, J=2.8Hz), 3.98(d, 1H, J=4.0Hz), 3.72-3.55(m, 2H), 3.3(t, 4H, J=6.6Hz), 1.97(s, 4H); HRMS (ESI+) m/z [M + H]⁺ calculated for C₂₁H₂₅N₇O₄: 440.2041; found: 440.2039.

### Example 12: Synthesis of (2R,3R,4S,5R)-2-{6-{2-[(E)-4-(1H-imidazol-1-yl)benzylidene] hydrazino}-9H-purin-9-yl}-5-(hydroxymethyl)tetrahydrofuran-3,4-diol (Compound 12)

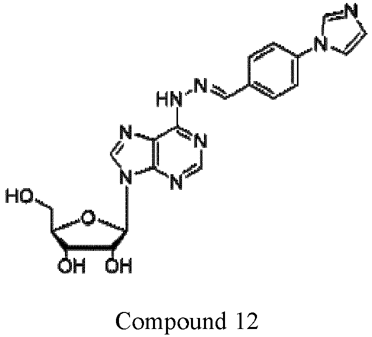

The method of step 1.2 in Example 1 was adopted, in which 4-(1H-imidazol-1-yl)benzaldehyde was used in place of pyrrole-2-carboxaldehyde to prepare Compound 12, and 683 mg white solid (Compound 12) was obtained. m.p. 222°C; ¹H NMR (DMSO-d₆): δ (ppm) 11.87(s, 1H), 8.57(s, 1H), 8.41(s, 1H), 8.38(s, 1H), 8.35(s, 1H), 7.90 (d, 2H, J=8.4Hz), 7.83(s, 1H), 7.76(d, 2H, J=8.8Hz), 7.14(s, 1H), 5.98(d, 1H, J=6.0Hz), 5.50(d, 1H, J=6.0Hz), 5.29(t, 1H, J=6.0Hz), 5.22(d, 1H, J=4.8Hz), 4.63(dd, 1H, J=5.2Hz, 5.6Hz), 4.19(dd, 1H, J=3.6Hz, 4.4Hz), 3.99(d, 1H, J=3.6Hz), 3.74-3.56(m, 2H); HRMS (ESI+) m/z [M + H]⁺ calculated for C₂₀H₂₀N₈O₄: 437.1680; found: 437.1714.

### Example 13: Synthesis of (2R,3S,4R,5R)-2-(hydroxymethyl)-5-{6-{2-[(E)-4-propoxybenzylidene]hydrazino}-9H- purin-9-yl}tetrahydrofuran-3,4-diol (Compound 13)

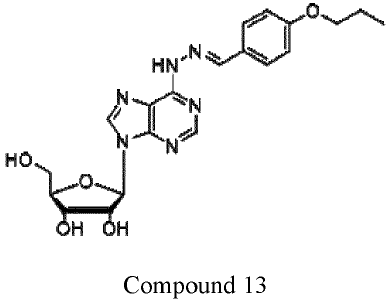

The method of step 1.2 in Example 1 was adopted, in which 4-propoxybenzaldehyde was used in place of pyrrole-2-carboxaldehyde to prepare Compound 13, and 686 mg of white solid (Compound 13) was obtained. m.p. 202°C; ¹H NMR (DMSO-d₆): δ (ppm) 11.67(s, 1H), 8.54(s, 1H), 8.38(s, 1H), 8.31(s, 1H), 7.68 (d, 2H, J=8.4Hz), 7.01(d, 2H, J=8.8Hz), 5.97(d, 1H, J=5.6Hz), 5.52(d, 1H,J=6.0Hz), 5.35(s, 1H), 5.24(d, 1H, J=4.8Hz), 4.63(d, 1H, J=4.8Hz), 4.18(d, 1H, J=3.2Hz), 3.99(s, 2H), 3.96(s, 1H), 3.72-3.57(m, 2H), 1.75(sext, 2H, J=7.2Hz, 6.8Hz, 6.4Hz), 1.00(t, 3H, J=7.6Hz); HRMS (ESI+) m/z [M + H]⁺ calculated for C₂₀H₂₄N₆O₅: 429.1881; found: 429.1881.

### Example 14: Synthesis of (2R,3S,4R,5R)-2-(hydroxymethyl)-5-{6-{2-[(E)-4-(trifluoromethyl) benzylidene]hydrazino}-9H-purin-9-yl}tetrahydrofuran-3,4-diol (Compound 14)

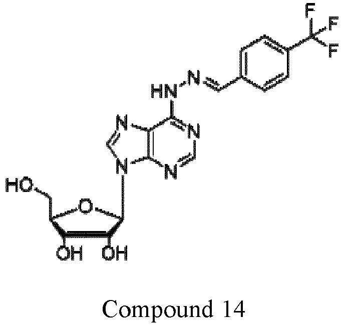

The method of step 1.2 in Example 1 was adopted, in which 4-(trifluoromethyl)benzaldehyde was used in place of pyrrole-2-carboxaldehyde to prepare Compound 14, and 639 mg of white solid was obtained. m.p. 182°C; ¹H NMR (DMSO-d₆): δ (ppm) 12.06(s, 1H), 8.60(s, 1H), 8.45(s, 1H), 8.42(s, 1H), 7.97 (d, 2H, J=8.4Hz), 7.82(d, 2H, J=8.8Hz), 5.99(d, 1H, J=5.6Hz), 5.54(d, 1H,J=5.6Hz), 5.31(t, 1H, J=5.2Hz), 5.26(d, 1H, J=4.8Hz), 4.63(dd, 1H, J=5.2Hz, 5.6Hz), 4.19(dd, 1H, J=3.6Hz, 4.4Hz), 3.96(d, 1H, J=3.6Hz), 3.73-3.59(m, 2H); HRMS (ESI+) m/z [M + H]⁺ calculated for C₁₈H₁₇F₃N₆O₄: 439.1336; found: 439.1336.

### Example 15: Synthesis of (2R,3R,4S,5R)-2-{6-{2-[(E)-(5-bromopyridin-2-yl)methylene] hydrazino}-9H-purin-9-yl}-5-(hydroxymethyl)tetrahydrofuran-3,4-diol (Compound 15)

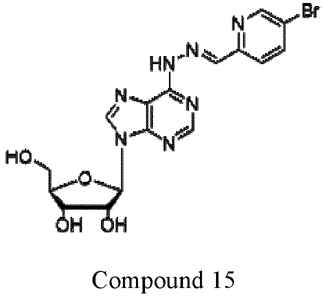

The method in step 1.2 of Example 1 was adopted, in which 5-bromo-2-pyridinaldehyde (5-bromopyridine-2-carbaldehyde) was used in place of pyrrole-2-carbaldehyde to prepare Compound 15, and 590 mg of yellow solid (Compound 15) was obtained. m.p. 211°C; ¹H NMR (DMSO-d₆): δ (ppm) 12.14(s, 1H), 8.72(s, 1H), 8.61(s, 1H), 8.45(s, 1H), 8.36(s, 1H), 8.15 (d, 1H, J=8.0Hz), 8.03(d, 1H, J=8.8Hz), 5.99(d, 1H, J=6.0Hz), 5.50(d, 1H,J=6.0Hz), 5.25(t, 1H, J=6.0Hz), 5.22(d, 1H, J=4.8Hz), 4.62(dd, 1H, J=5.2Hz, 5.6Hz), 4.19(dd, 1H, J=3.6Hz, 4.8Hz), 3.99(d, 1H, J=3.2Hz), 3.73-3.56(m, 2H); HRMS (ESI+) m/z [M + H]⁺ calculated for C₁₆H₁₆BrN₇O₄: 450.0520; found: 450.0520.

### Example 16: Synthesis of (2R,3S,4R,5R)-2-(hydroxymethyl)-5-{6-{2-[(E)-thiazol-5-yl-methylene]hydrazino}-9H-purin-9-yl}tetrahydrofuran-3,4-diol (Compound 16)

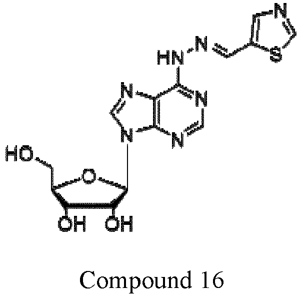

The method in step 1.2 of Example 1 was obtained, in which thiazole-5-formaldehyde (1,3-thiazole-5-carbaldehyde) was used in place of pyrrole-2-carbaldehyde to prepare Compound 16, and 440 mg of white solid (Compound 16) was obtained. m.p. 224°C; ¹H NMR (DMSO-d₆): δ (ppm) 12.00(s, 1H), 9.13(s, 1H), 8.65(s, 1H), 8.56(s, 1H), 8.42(s, 1H), 8.20 (s, 1H), 5.96(d, 1H, J=6.0Hz), 5.52(d, 1H,J=6.0Hz), 5.30(s, 1H), 5.24(d, 1H, J=4.8Hz), 4.62(dd, 1H, J=5.2Hz, 5.6Hz), 4.17(dd, 1H, J=3.6Hz), 3.98(d, 1H, J=3.6Hz), 3.72-3.55(m, 2H); HRMS (ESI+) m/z [M + H]⁺ calculated for C₁₄H₁₅N₇O₄S: 378.0979; found: 378.0978.

### Example 17: Synthesis of (2R,3R,4S,5R)-2-{6-{2-{(1E,2E)-3-[4-(dimethylamino)phenyl] allylidene}hydrazino}-9H-purin-9-yl}-5-(hydroxymethyl)tetrahydrofuran-3,4-diol (Compound 17)

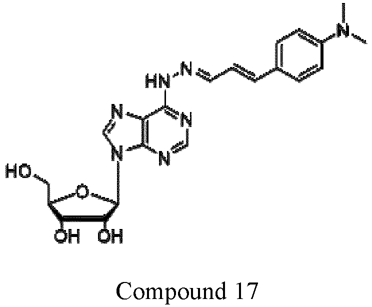

The method in step 1.2 of Example 1 was adopted, in which 4-dimethylamino-cinnamaldehyde ((2E)-3-[4-(dimethylamino)phenyl]prop-2-enal) was used in place of pyrrole-2-carboxaldehyde to prepare Compound 17, and 490 mg of yellow solid (Compound 17) was obtained. m.p. 172°C; ¹H NMR (DMSO-d₆): δ (ppm) 11.57(br, 1H), 8.51(d, 1H, J=3.6Hz), 8.37(d, 1H, J=6.0Hz), 8.16(br, 1H), 7.44(d, 1H, J=8.4Hz), 7.31(d, 1H, J=8.8Hz), 6.83-6.70(m, 4H), 5.95(d, 1H, J=5.6Hz), 5.52(d, 1H,J=6.0Hz), 5.35(dd, 1H, J=5.2Hz, 6.0Hz), 5.24(d, 1H, J=4.4Hz), 4.63(dd, 1H, J=4.8Hz, 6.0Hz), 4.17(d, 1H, J=3.6Hz), 3.98(d, 1H, J=3.2Hz), 3.71-3.56(m, 2H), 2.95(s, 6H); HRMS (ESI+) m/z [M + H]⁺ calculated for C₂₁H₂₅N₇O₄: 440.2041; found: 440.2044.

### Example 18: Synthesis of (2R,3R,4S,5R)-2-{6-{2-[(E)-4-chloro-3-(trifluoromethyl)benzylidene] hydrazino}-9H-purin-9-yl}-5-(hydroxymethyl)tetrahydrofuran-3,4-diol (Compound 18)

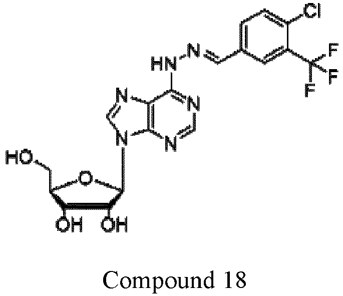

The method of step 1.2 in Example 1 was adopted, in which 4-chloro-3-(trifluoromethyl)benzaldehyde was used in place of pyrrole-2-carboxaldehyde to prepare Compound 18, and 600mg of white solid was obtained. m.p. 196°C; ¹H NMR (DMSO-d₆): δ (ppm) 12.10(s, 1H), 8.60(s, 1H), 8.45(s, 1H), 8.40(s, 1H), 8.22 (s, 1H), 8.06(d, 1H, J=8.4Hz), 7.82(d, 1H, J=8.4Hz), 5.99(d, 1H, J=6.0Hz), 5.53(d, 1H,J=5.6Hz), 5.29(t, 1H, J=5.6Hz), 5.25(d, 1H, J=4.8Hz), 4.63(dd, 1H, J=5.2Hz, 6.0Hz), 4.19(dd, 1H, J=3.6Hz, 4.8Hz), 3.99(d, 1H, J=3.6Hz), 3.73-3.56(m, 2H); HRMS (ESI+) m/z [M + H]⁺ calculated for C₁₈H₁₆ClF₃N₆O₄: 473.0946; found: 473.0945.

### Example 19: Synthesis of (2R,3R,4S,5R)-2-{6-{2-[(E)-4-(diphenylamino)benzylidene] hydrazino}-9H-purin-9-yl}-5-(hydroxymethyl)tetrahydrofuran-3,4-diol (Compound 19)

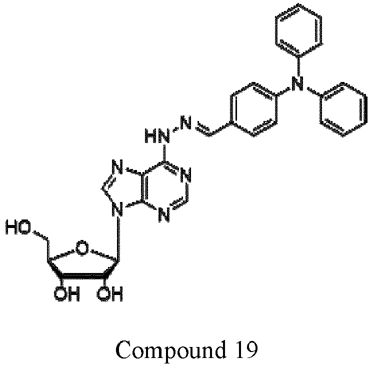

The method of step 1.2 in Example 1 was obtained, in which 4-(N,N-diphenylamino)benzaldehyde (4-(diphenylamino)benzaldehyde) was used in place of pyrrole-2-carbaldehyde to prepare Compound 19, and 460 mg of white solid (Compound 19) was obtained. m.p. 160°C; ¹H NMR (DMSO-d₆): δ (ppm) 11.66(s, 1H), 8.51(s, 1H), 8.37(s, 1H), 8.31(s, 1H), 7.63(d, 2H, J=8.4Hz), 7.35(t, 4H, J=8.0Hz), 7.13-7.07(m, 6H), 6.99(d, 2H, J=8.8Hz), 5.96(d, 1H, J=6.0Hz), 5.48(d, 1H,J=6.0Hz), 5.31(t, 1H, J=6.0Hz), 5.20(d, 1H, J=4.4Hz), 4.62(dd, 1H, J=5.2Hz, 5.6Hz), 4.17(dd, 1H, J=3.6Hz, 4.8Hz), 3.98(d, 1H, J=3.2Hz), 3.72-3.55(m, 2H); HRMS (ESI+) m/z [M + H]⁺ calculated for C₂₉H₂₇N₇O₄: 538.2197; found: 538.2198.

### Example 20: Synthesis of (2R,3R,4S,5R)-2-{6-{2-[(E)-(2-butyl-5-chloro-1H-imidazol-4-yl) methylene]hydrazino}-9H-purin-9-yl}-5-(hydroxymethyl)tetrahydrofuran-3,4-diol (Compound 20)

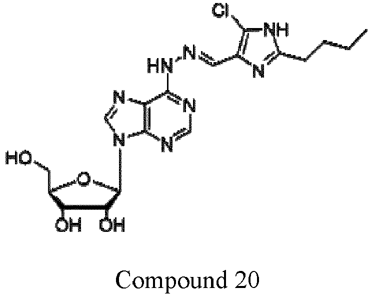

The method in step 1.2 of Example 1 was adopted, in which 2-butyl-5-chloro-1H-imidazole-4-carbaldehyde was used in place of pyrrole-2-carbaldehyde to prepare Compound 20, and 640 mg of white solid (Compound 20) was obtained. m.p. 178°C; ¹H NMR (DMSO-d₆): δ (ppm) 12.79(s, 1H), 11.78(s, 1H), 8.53(s, 1H), 8.38(s, 1H), 8.36(s, 1H), 5.96(d, 1H, J=6.0Hz), 5.53(d, 1H,J=6.4Hz), 5.36(s, 1H), 5.26(d, 1H, J=4.4Hz), 4.64(dd, 1H, J=5.2Hz, 5.6Hz), 4.17(d, 1H, J=3.6Hz), 3.99(d, 1H, J=2.8Hz), 3.71-3.56(m, 2H), 2.66(t, 2H, J=7.6Hz), 1.62(quint, 2H, J=7.6Hz,7.2Hz), 1.29(sext, 2H, J=7.6Hz, 7.6Hz, 7.2Hz), 0.891(t, 3H, J=7.2Hz); HRMS (ESI+) m/z [M + H]⁺ calculated for C₁₈H₂₃ClN₈O₄: 451.1604; found: 451.1606.

### Example 21: Synthesis of (2R,3R,4S,5R)-2-{6-{2-[(1E,2E,4E)-deca-2,4-dien-1-ylidene] hydrazino}-9H-purin-9-yl}-5-(hydroxymethyl)tetrahydrofuran-3,4-diol (Compound 21)

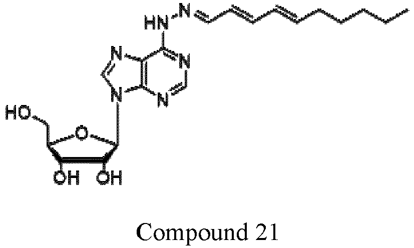

The method of step 1.2 in Example 1 was adopted, in which (2E,4E)-deca-2,4-dienal was used in place of pyrrole-2-carboxaldehyde to prepare Compound 21, and 112 mg of white solid (Compound 21) was obtained. m.p. 178°C; ¹H NMR (DMSO-d₆): δ (ppm) 11.59(s, 1H), 8.50(s, 1H), 8.33(s, 1H), 8.04(d, 1H, J=9.6Hz), 6.61-6.00(m, 3H), 5.93(d, 1H, J=5.6Hz), 5.50(d, 1H,J=6.4Hz), 5.32(t, 1H, J=4.8Hz), 5.23(d, 1H, J=4.8Hz), 4.61(dd, 1H, J=5.2Hz, 6.0Hz), 4.16(d, 1H, J=3.6Hz), 3.97(d, 1H, J=3.2Hz), 3.71-3.54(m, 2H), 2.13(dd, 1H, J=6.8Hz, 7.2Hz), 1.43-1.23(m, 8H), 0.88(t, 3H, J=6.8Hz); HRMS (ESI+) m/z [M + H]⁺ calculated for C₂₀H₂₈N₆O₄: 417.2245; found: 417.2245.

### Example 22: Synthesis of (2R,3R,4S,5R)-2-{6-{2-[(E)-cyclohexylmethylene]hydrazino}-9H-purin-9-yl}-5-(hydroxyl methyl)tetrahydrofuran-3,4-diol (Compound 22)

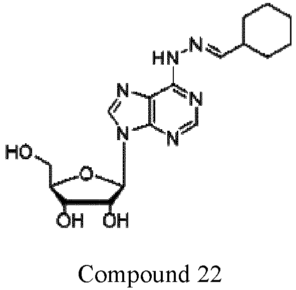

The method of step 1.2 in Example 1 was adopted, in which cyclohexanecarbaldehyde was used in place of pyrrole-2-carbaldehyde to prepare Compound 22, and 300 mg of white solid (Compound 22) was obtained. m.p. 132°C; ¹H NMR (DMSO-d₆): δ (ppm) 11.25(s, 1H), 8.46(s, 1H), 8.30(s, 1H), 7.60(d, 1H, J=4.8Hz), 5.93(d, 1H, J=6.4Hz), 5.50(d, 1H,J=6.0Hz), 5.35(s, 1H), 5.23(d, 1H, J=4.4Hz), 4.61(dd, 1H, J=5.6Hz, 5.6Hz), 4.16(d, 1H, J=3.2Hz, 4.4Hz), 3.97(dd, 1H, J=3.2Hz, 3.6Hz), 3.70-3.54(m, 2H), 2.27(d, 1H, J=4.8Hz), 1.80-1.62(m, 5H), 1.35-1.18(m, 5H); HRMS (ESI+) m/z [M + H]⁺ calculated for C₁₇H₂₄N₆O₄: 377.1932; found: 377.1934.

### Example 23: Radioligand binding test

### 1) Experimental materials

[3H]CGS21680 (2-[p-(2-carboxyethyl)phenylethylamino]-5'-N-ethylformamidoadenosine, [carboxy-1-ethyl-3H(N)]-; 250µCi) was purchased from PerkinElmer Research Products (Boston, MA).

Cell membrane stably transfected with (human) A_{2A} adenosine receptor was prepared in HEK-293 cells. The cell membrane was obtained from PerkinElmer Research Products (Boston, MA).

CGS21680 (2-[p-(2-carboxyethyl)phenylethylamino]-5'-N-ethylformamidoadenosine) was purchased from Selleck (Shanghai, CN).

All other reagents were of analytical grade and obtained from commercial sources.

### 2) Experimental method

The A_{2A} adenosine receptors used were all expressed in the cell membrane. The compound was diluted 3 times serially with DMSO (Solarbio, D8371-250ml) so as to generate a compound source plate with 10 different concentrations (10µM, 3.3µM, 1.1µM, 0.37µM, 0.12µM, 0.0412µM, 0.0137µM, 0.0046µM, 0.0015µM, 0.0005µM); 250 nL of the compound was added to a 384-well Opti-plate, sealed with parafilm; to 1 mL of detection buffer (50 mM Tris-HCl pH 7.4, 10 mM MgCl₂, 1 mM EDTA, 1µg/mL adenosine deaminase), 20 U of hA_{2A} HEK-293 cell membrane was added for dilution; to the diluted cell membrane, 0.75 µCi [3H]CGS 21680 (final 25 nM) was added and mixed well; 50 µL of the prepared cell membrane diluent was transferred to a 384-well Opti-plate containing a new compound, and incubated at 25 °C for 90 minutes; to a UNIFILTER-96 GF/B filter plate, 100 µL of 0.5% polyethyleneimine solution (PEI) was added to soak at 4°C for 90 min; then Cell Harvester was used to transfer 500 µL of washing buffer/well (50mM Tris-HCl pH 7.4, 154mM NaCl), and the UNIFILTER-96 GF/B filter plate was washed twice; the mixture system in the Opti-plate was transferred to the washed UNIFILTER-96 GF/B filter plate; 500 µL of washing buffer/well (50mM Tris-HCl pH 7.4, 154mM NaCl) was used to wash the UNIFILTER-96 GF/B filter plate 9 times; incubation was performed in a 37°C incubator for 3min; 40 µL of ULTIMA GOLD scintillation solution (Perkin Elmer, Cat #77-16061) was added to each well, and MicroBeta liquid scintillation counter (PerkinElmer) was used to read CPM (count per minute) value. The specific binding percentage of [3H]CGS21680 was calculated according to the CPM value, % specific binding of [3H]CGS21680 = (CPMₛₐₘₚₗₑ - CPM_{Low Control}) / (CPM_{High control} - CPM_{Low Control}) ^{∗} 100, in which High Control was 0.5% DMSO, Low Control was 100 µM CGS21680. The IC₅₀ value was calculated based on the compound concentration and the specific binding percentage of [3H]CGS21680 by curve fitting.

### 3) Experimental results

The inhibition constant (Kᵢ) value was calculated from the IC₅₀ value according to the Cheng and Prusoff equation, Kᵢ= IC₅₀/(1+[S]/Kₘ), in which [S] was the concentration of the radioligand (25nM), and Kₘ was the human A_{2A}AR dissociation constant (22 nM) of [3H]CGS21680. The inhibition constant Kᵢ values for Compounds 1 to 20 of the present invention binding to A_{2A} adenosine receptor were shown in Table 1.

**Table 1: Binding test results of compounds and A_{2A} adenosine receptor**

| Compound | Kᵢ (nM) | Compound | Kᵢ (nM) |
|---|---|---|---|
| Compound 1 | 945.1 | Compound 12 | 15.4 |
| Compound 2 | 2147 | Compound 13 | 4470 |
| Compound 3 | 3.6 | Compound 14 | 520 |
| Compound 4 | >10,000 | Compound 15 | 3.4 |
| Compound 5 | 353 | Compound 16 | 15.8 |
| Compound 6 | 1.2 | Compound 17 | 1.4 |
| Compound 7 | 13.8 | Compound 18 | 1466 |
| Compound 8 | 236 | Compound 19 | 5581 |
| Compound 9 | 4593 | Compound 20 | 998.4 |
| Compound 10 | 1903 | Compound 21 | 1.2 |
| Compound 11 | 2.0 | Compound 22 | 86.6 |

### Example 24: Adenosine receptor A_{2A} cAMP test

### 1) Experimental materials

Experimental reagents and consumables: DMEM/F12, G418, Penicillin-Streptomycin, Versene Solution, HEPES, Hank's Buffered Saline Solution, PBS (pH 7.4, 1×, sterile), FBS, BSA Stabilizer 7.5%, Rolipram, NECA, were purchased from Gibico, Hyclone and Sigma, respectively. LANCE^{®} Ultra cAMP kit (Eu-cAMP tracer, Ulight-anti-cAMP reagent, cAMP detection buffer) and hADORA_{2A}-HEK293 cells were purchased from PerkinElmer Research Products (Boston, MA). All other reagents were of analytical grade and obtained from commercial sources. 384-well polypropylene microplate and 384-well white solid plate were purchased from Labcyte and Corning, respectively.

Experimental instruments: TECAN automated pipetting workstation, Echo ultrasonic pipetting system, and EnVison microplate reader were purchased from TECAN, Labcyte and Envision, respectively.

### 2) Experimental method

Cells stably expressing human adenosine receptor A_{2A} (hADORA_{2A}-HEK293 cells) were cultured in DMEM/F12 medium containing 10% FBS, 1X Penicillin-Streptomycin and 400µg/ml G418 in a 37°C, 5% CO₂ environment. Before the experiment, the cells were digested with Versene solution, and the cells were collected by centrifugation at 200g at room temperature for 5 minutes, and finally resuspended with detection buffer (Hank's buffered saline solution, containing 5mM HEPES, 0.1% BSA stabilizer and 10µM Rolipram, pH 7.4). TECAN automated pipetting workstation was used to prepare a compound source plate by 3-fold diluting the compound in a 384-well polypropylene microplate with DMSO to form 11 concentration points, in which the 11 concentration points of the compound were 10mM, 3.33mM, 1.11mM, 0.37mM, 0.12mM, 0.041mM, 0.013mM, 4.57×10⁻³mM, 1.52×10⁻³mM, 5×10⁻⁴mM and 1.7×10⁻⁴mM, respectively. Echo ultrasonic pipetting system (Labcyte) was used to transfer the test compound from the compound source plate to the detection plate, in which the volume of the compound transferred was 10 nl/well. The hADORA_{2A}-HEK293 cell suspension was diluted with detection buffer to 30,000 cells/ml, and the cell suspension was transferred to the detection plate at a volume of 10 µl/well (300 cells/well). The detection plate was centrifuged at 150g for 1 minute and preincubated at room temperature for 30 minutes. Eu-cAMP tracer working solution (40µl of Eu-cAMP tracer, 1.96ml of cAMP detection buffer) was added to the detection plate (5µl/well), and then Ulight-anti-cAMP working solution (13µl of Ulight-anti-cAMP reagent, and 1.95ml of cAMP detection buffer) was added to the detection plate (5µl/well). The detection plate was rotated at 150g for 30 seconds, and incubated at room temperature for 30 minutes. EnVison microplate reader (EnVision multimode plate reader, PerkinElmer) was used to test the level of cyclic adenosine monophosphate in the final solution (λₑₓ=320 nm, λₑₘ=665 nm & 615 nm). The EC₅₀ (nM) value of the compound interacting with A_{2A} adenosine receptor to stimulate the production of a level of cyclic adenosine monophosphate was calculated. The compound A_{2A} receptor agonist titer was expressed as the EC₅₀ (nM) value of the compound interacting with the A_{2A} adenosine receptor to stimulate the production of a level of cyclic adenosine monophosphate.

### 3) Experimental results

The EC₅₀ (nM) values of the test compounds interacting with A_{2A}AR to stimulate AMP level were shown in Table 2. The results showed that Compounds 7, 15 and 16 prepared by the present invention were all hA_{2A}AR agonists. When Compounds 7, 15 and 16 interacted with A_{2A}AR, their inhibition constant Kᵢ values and EC₅₀ values of stimulating cAMP were basically in the same nanomolar range.

**Table 2: Results of EC₅₀ values of A_{2A} agonist function determination of compounds**

| Compound | cAMP EC₅₀ (nM) |
|---|---|
| Compound 7 | 18.6 |
| Compound 15 | 7.3 |
| Compound 16 | 43.5 |

### Example 25: Animal experiment of blood-brain barrier opening method

### 1) Experimental materials

Fluorescein-labeled dextran FITC-Dextran (CAS: 60842-46-8) with a molecular weight of 10,000 MW was purchased from Tixiai (Shanghai) Chemical Industry Development Co., Ltd.; PBS solution and experimental animal SD rats were obtained from commercial sources.

### 2) Experimental method

FITC-Dextran solution was prepared with PBS to obtain six concentration gradients (0.001, 0.01, 0.1, 1, 0.5, 10 µg/ml), and a FITC-Dextran concentration standard curve was prepared by using microplate reader (λₑₓ=490 nm, λₑₘ= 520 nm); 10mg/ml FITC-Dextran solution was separately prepared, Compound 5 was added to PBS solution to make 1mg/ml solution, 1ml of 10mg/ml FITC-Dextran solution and 1ml of 1mg/ml Compound 5 PBS solution were taken to make an administration solution; 1ml of 10mg/ml FITC-Dextran solution and 1ml of PBS solution taken to make a blank control solution; 6 SD rats were injected with 2ml of the administration solution respectively in the tail vein, while another 6 SD rats were injected with 2ml of the blank control solution in the tail vein; after 30 minutes, the brain tissues of all SD rats were taken out, homogenized and centrifuged at 10,000 rpm for 15 minutes, and the supernatants were taken for testing; and a microplate reader (λₑₓ=490 nm, λₑₘ=520 nm) was used for the fluorescence detection of the solutions to be tested.

### 3) Experimental results

The fluorescence values measured by the microplate reader were converted into the corresponding FITC-Dextran average concentrations according to the obtained FITC-Dextran concentration standard curve. The results were shown in Table 3. The results showed that the macromolecule FITC-Dextran itself could pass through the blood-brain barrier, while the FITC-Dextran added with Compound 5 could enter the brain through the BBB, indicating that Compound 5 could open the blood-brain barrier.

**Table 3: Results of FITC-Dextran concentration detection in the brain of SD rats**

| | PBS solution containing Compound 7 | Blank control solution |
|---|---|---|
| FITC-Dextran concentration (µg/ml) | 0.057 | 0.021 |

Although the specific embodiments of the present disclosure have been described in details, those skilled in the art will understand that according to all the teachings that have been disclosed, various modifications and substitutions can be made to those details, and these changes are within the protection scope of the present disclosure. The full scope of the disclosure is given by the appended claims and any equivalents thereof. The publications and patent documents cited in this disclosure are incorporated herein by reference.

## Claims

1. A compound represented by the general Formula (I), or a stereoisomer thereof, or a pharmaceutically acceptable salt of the compound or stereoisomer, or a pharmaceutically acceptable hydrate or solvate of the compound or stereoisomer, or a pharmaceutically acceptable ester of the compound or stereoisomer, wherein the compound has a structure represented by the general Formula (I): 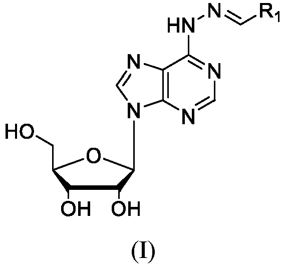 wherein,
R₁ is selected from the group consisting of aryl, heteroaryl, cycloalkyl, C₁₋₁₀ alkyl, heterocycloalkyl, C₁₋₁₀ heteroalkyl or C₂₋₁₀ alkenyl;
R₁ is optionally substituted with one or more R', each R' is independently selected from the group consisting of phenyl, halophenyl, amino-substituted phenyl, benzyloxy, halobenzyloxy, phenylamino, heteroaryl, cycloalkyl, heterocycloalkyl, C₁₋₆ alkyl, halogenated C₁₋₆ alkyl, C₂₋₆ alkenyl, C₁₋₆ alkoxy, C₁₋₆ alkylthio, -NHC(O)R¹⁰, halogen or cyano, wherein R¹⁰ is C₁₋₆ alkyl.

2. The compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to claim 1, wherein:
R₁ is selected from C₆₋₁₀ aryl, 5- to 7-membered heteroaryl, 5- to 6-membered cycloalkyl, 5-to 6-membered heterocycloalkyl, C₁₋₁₀ alkyl, C₁₋₁₀ heteroalkyl or C₂₋₁₀ alkenyl;
preferably, R₁ is selected from the group consisting of phenyl, pyrrolyl, imidazolyl, thiazolyl, furyl, pyridyl, cyclopentyl, cyclohexyl, methyl, ethyl, n-propyl, isopropyl, n-butyl, isobutyl, tert-butyl, trifluoromethyl, methoxy, ethoxy, n-propoxy, isopropoxy, n-butoxy, tert-butoxy, sec-butoxy, n-pentyloxy, n-hexyloxy, methylthio, ethylthio, n-propylthio, isopropylthio, n-butylthio, tert-butylthio, sec-butylthio, n-pentylthio, n-hexylthio or C₂₋₁₀ alkenyl;
preferably, R₁ is selected from the group consisting of phenyl, pyrrolyl, furyl, imidazolyl, thiazolyl, cyclohexyl, alkylthio, methyl, ethyl, n-propyl, isopropyl, n-butyl, isobutyl, tert-butyl, n-pentyl, tert-pentyl, neopentyl, hexyl, trifluoromethyl, difluoromethyl, fluoromethyl, vinyl, or decadienyl.

3. The compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to claim 1, wherein:
each R' is independently selected from the group consisting of phenyl, halophenyl, amino-substituted phenyl, benzyloxy, halobenzyloxy, phenylamino, imidazolyl, pyridyl, 5- to 6-membered cycloalkyl, 5- to 6-membered heterocycloalkyl, C₁₋₆ alkyl, C₁₋₆ haloalkyl, -NHC(O)R¹⁰, halogen or cyano, wherein R¹⁰ is C₁₋₄ alkyl;
preferably, each R' is independently selected from the group consisting of phenyl, halophenyl, dimethylamino-substituted phenyl, benzyloxy, halobenzyloxy, diphenylamino, 1H-imidazol-1-yl, pyridin-2-yl, 1H-imidazol-1-yl, pyrrolidin-1-yl, cyclopentyl, cyclohexyl, methyl, ethyl, n-propyl, isopropyl, n-butyl, isobutyl, tert-butyl, trifluoromethyl, methoxy, ethoxy, n-propoxy, isopropoxy, n-butoxy, tert-butoxy, sec-butoxy, n-pentyloxy, n-hexyloxy, methylthio, ethylthio, n-propylthio, isopropylthio, n-butylthio, tert-butylthio, sec-butylthio, n-pentylthio, n-hexylthio, -NH(CO)CH₃, F, Cl, Br or cyano.

4. The compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to claim 1, wherein the compound has a structure represented by Formula I-1: 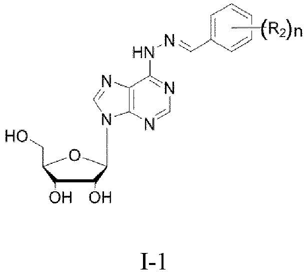
R₂ represents a substituent attached to the benzene ring;
n is 1, 2, 3, 4 or 5;
each R₂ is independently selected from the group consisting of phenyl, halophenyl, amino-substituted phenyl, benzyloxy, halobenzyloxy, phenylamino, heteroaryl, cycloalkyl, heterocycloalkyl, C₁₋₆ alkyl, C₁₋₆ heteroalkyl, C₁₋₆ haloalkyl, C₂₋₁₀ alkenyl (e.g., C₂₋₆ alkenyl), C₁₋₆ alkoxy, C₁₋₆ haloalkoxy, C₁₋₆ alkylamino, acylamino, halogen, hydroxy, cyano or -NHC(O)R¹⁰, wherein R¹⁰ is C₁₋₄ alkyl;
preferably, each R₂ is independently selected from the group consisting of phenyl, halophenyl, amino-substituted phenyl, benzyloxy, halobenzyloxy, phenylamino, heteroaryl, cycloalkyl, heterocycloalkyl, C₁₋₆ alkyl, C₁₋₆ haloalkyl, C₂₋₁₀ alkenyl (e.g., C₂₋₆ alkenyl), C₁₋₆ alkoxy, - NHC(O)R¹⁰, halogen or cyano, wherein R¹⁰ is C₁₋₄ alkyl.

5. The compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to claim 4, wherein,
each R₂ is independently selected from the group consisting of C₁₋₆ alkyl, C₁₋₆ heteroalkyl, C₁₋₆ haloalkyl, C₁₋₆ alkoxy, C₁₋₆ haloalkoxy, C₁₋₆ alkylamino, acylamino, phenyl, benzyloxy, halobenzyloxy, phenylamino, 5- to 6-membered heterocycloalkyl, -NH(CO)CH₃, halogen, hydroxy, or cyano.

6. The compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to claim 4, wherein,
each R₂ is independently selected from the group consisting of methyl, trifluoromethyl, ethyl, n-propyl, isopropyl, n-butyl, isobutyl, tert-butyl, C₁₋₃ alkoxy, phenyl, diphenylamino, benzyloxy, halobenzyloxy, pyridin-2-yl, 1H-imidazol-1-yl, pyrrolidin-1-yl, -NH(CO)CH₃, F, Cl, Br or cyano.

7. The compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to claim 1, wherein the compound is selected from:

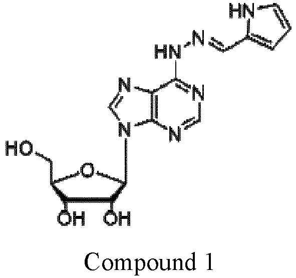

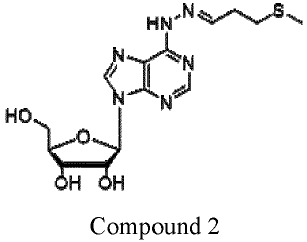

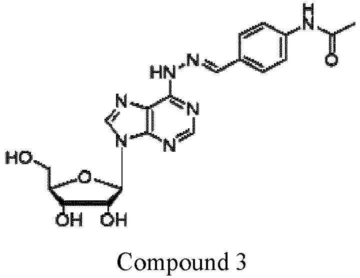

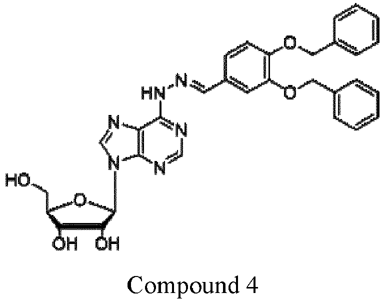

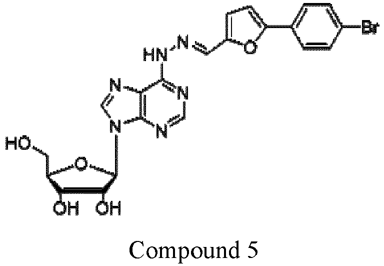

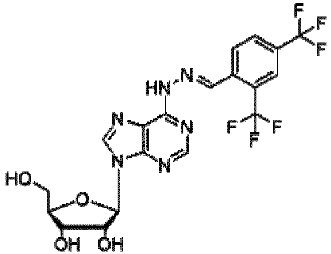

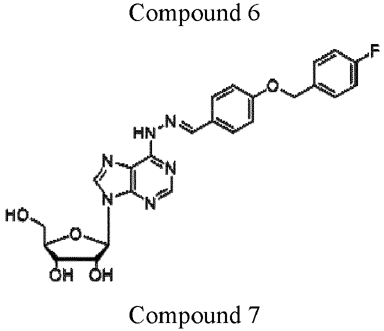

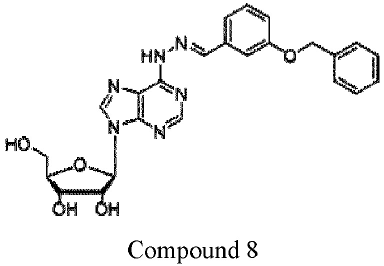

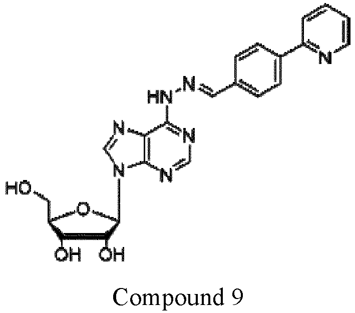

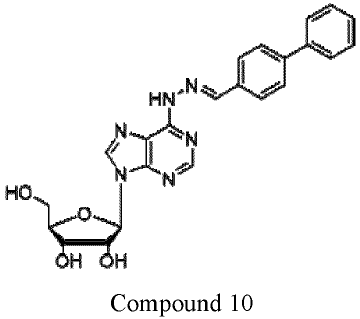

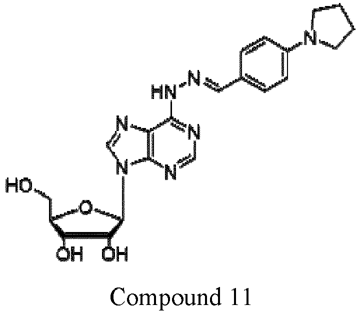

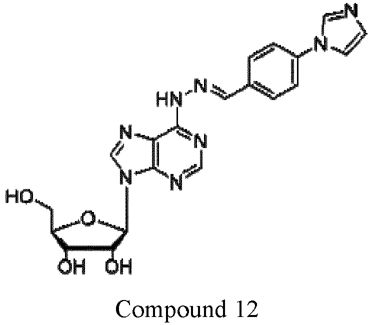

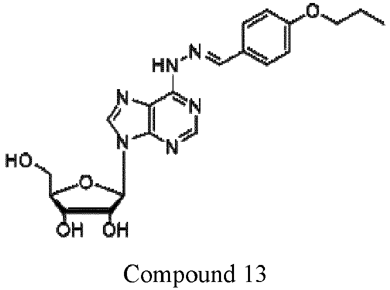

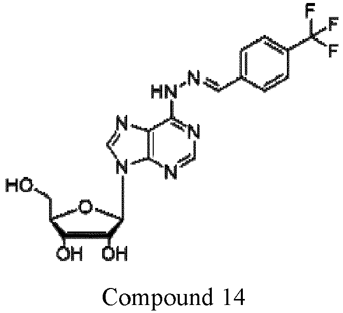

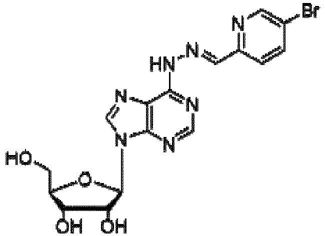

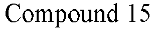

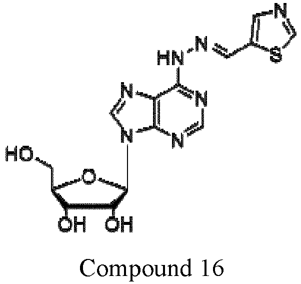

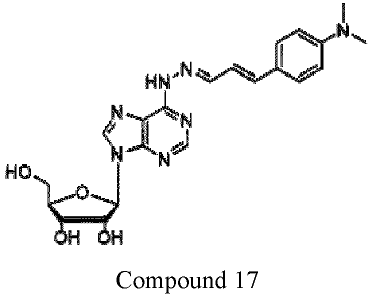

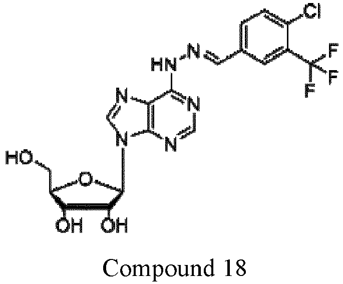

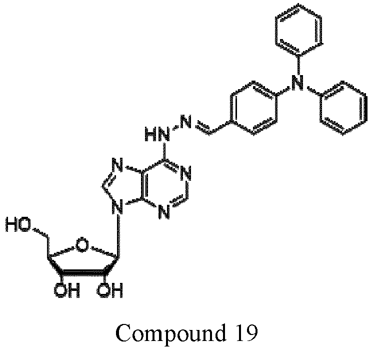

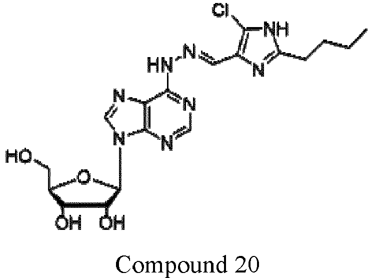

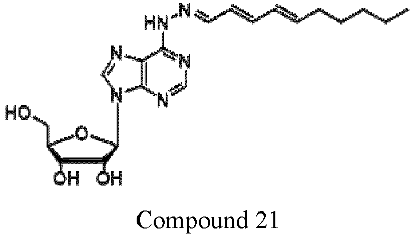

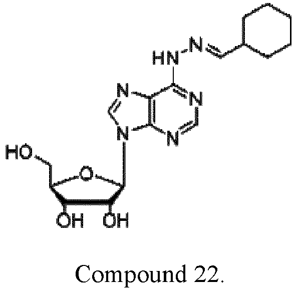

8. A method for preparing the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, comprising: 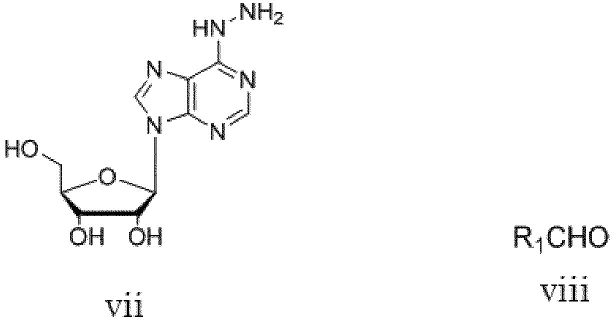 reacting a compound of Formula (vii) with a substituted formaldehyde represented by Formula (viii) to obtain the compound represented by general Formula (I), wherein the definition of R₁ is the same as that described in any one of claims 1 to 3;
preferably, the compound of Formula (vii) reacts with the substituted formaldehyde (viii) in a methanol solution under a microwave at 70~90°C; 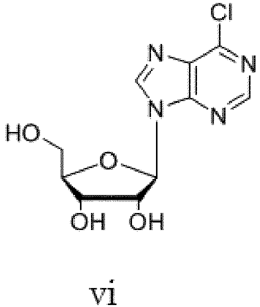 preferably, the compound of Formula (vii) is produced from a compound of Formula (vi) by hydrazinolysis with hydrazine hydrate at 60~80°C.

9. A pharmaceutical composition, which comprises at least one of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, and one or more pharmaceutically acceptable carriers or excipients.

10. The pharmaceutical composition according to claim 9, which further comprises:
a drug for crossing the blood-brain barrier, which is selected from the group consisting of a drug for treating a disease or disorder of the central nervous system, a neurotoxin antidote, and a drug for treating a brain glioma.

11. Use of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, or the pharmaceutical composition according to claim 9 or 10 in the manufacture of a medicament as an A_{2A} adenosine receptor agonist, or
in the manufacture of a medicament for the prevention and/or treatment of a human pathological condition or symptom, wherein the prevention or treatment of a human pathological condition or symptom is related to the activity of A_{2A} adenosine receptor, and the prevention and/or treatment of a human pathological condition or symptom requires agonizing of the A_{2A} adenosine receptor.

12. Use according to claim 11, wherein the human pathological condition or symptom is selected from the group consisting of: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, founder's disease, glaucoma, ocular hypertension, spinal cord injury, myocardial infarction, and acute myocardial infarction.

13. Use of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, or the pharmaceutical composition according to claim 9 or 10 in the manufacture of a medicament for diagnosing a human myocardial perfusion abnormality.

14. Use of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, or the pharmaceutical composition according to claim 9 or 10 in the manufacture of a medicament for increasing a blood-brain barrier permeability of a subject receiving a therapeutic drug, wherein the subject is benefited from the increased blood-brain barrier permeability for delivering the therapeutic drug across the blood-brain barrier.

15. Use according to claim 14, wherein the therapeutic drug is selected from the group consisting of: a drug that is effective in treating a disease or disorder of the central nervous system, a neurotoxin antidote, and a drug for treating a brain glioma.

16. A method for prevention and/or treatment of a human pathological condition or symptom, comprising administering to a patient in need of such treatment a therapeutically effective amount of at least one of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, or the pharmaceutical composition according to claim 9 or 10, wherein the human pathological condition or symptom is related to the activity of A_{2A} adenosine receptor, and the prevention or treatment of the pathological condition or symptom of the patient requires agonizing of the A_{2A} adenosine receptor;
preferably, the human pathological condition or symptom is selected from the group consisting of: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, founder's disease, glaucoma, ocular hypertension, spinal cord injury, myocardial infarction, and acute myocardial infarction.

17. The compound represented by the general Formula (I), or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, for use in prevention and/or treatment of a human pathological condition or symptom, the human pathological condition or symptom is related to the activity of A_{2A} adenosine receptor, and the prevention or treatment of the human pathological condition or symptom requires agonizing of the A_{2A} adenosine receptor;
preferably, the human pathological condition or symptom is selected from the group consisting of: autoimmune irritation, inflammation, allergic disease, skin disease, infectious disease, wasting disease, neuropathic pain, open trauma, adverse reaction caused by drug therapy, cardiovascular disease, ischemia-reperfusion injury, gout, chemical trauma, thermal trauma, diabetic nephropathy, sickle cell disease, laminitis, founder's disease, glaucoma, ocular hypertension, spinal cord injury, myocardial infarction, and acute myocardial infarction.

18. The compound represented by the general Formula (I), or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, for use in agonizing A_{2A} adenosine receptor or vasodilating a coronary artery, or
for use in diagnosing a human myocardial perfusion abnormality, or
for use in increasing a blood-brain barrier permeability of a subject receiving a therapeutic drug, in which the subject benefits from the increased blood-brain barrier permeability for delivering the therapeutic drug across the blood-brain barrier,
preferably, the therapeutic drug is selected from the group consisting of: a drug for treating a disease or disorder of the central nervous system, a neurotoxin antidote, and a drug for treating a brain glioma.

19. A method for diagnosing a human myocardial perfusion abnormality, comprising administering to a patient in need of such diagnosis a diagnostically effective amount of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, or the pharmaceutical composition according to claim 9 or 10.

20. A method for increasing a blood-brain barrier permeability of a subject receiving a therapeutic drug, the method comprising administering to the subject an effective amount of the compound, or the stereoisomer thereof, or the pharmaceutically acceptable salt of the compound or stereoisomer, or the pharmaceutically acceptable hydrate or solvate of the compound or stereoisomer, or the pharmaceutically acceptable ester of the compound or stereoisomer, according to any one of claims 1 to 7, or the pharmaceutical composition according to claim 9 or 10, wherein the subject benefits from the increased blood-brain barrier permeability for delivering the therapeutic drug across the blood-brain barrier;
preferably, the therapeutic drug is selected from the group consisting of: a drug for treating a disease or disorder of the central nervous system, a neurotoxin antidote, and a drug for treating a brain glioma.
